(19) Europäisches Patentamt
European Patent Office
Office européen des brevets

(11) EP 1 034 164 B1

(12) # EUROPEAN PATENT SPECIFICATION

(45) Date of publication and mention of the grant of the patent:
**19.05.2004 Bulletin 2004/21**

(21) Application number: **98959547.5**

(22) Date of filing: **24.11.1998**

(51) Int Cl.$^7$: **C07D 207/16**, C07D 207/48, C07D 211/34, C07D 211/96, C07D 217/26, C07D 241/04, C07D 401/12, C07D 403/12, C07D 405/12, C07D 413/12, A61K 31/40, A61K 31/47, A61K 31/495

(86) International application number:
**PCT/US1998/024898**

(87) International publication number:
**WO 1999/026921 (03.06.1999 Gazette 1999/22)**

(54) **SUBSTITUTED BETA-ALANINE DERIVATIVES AS CELL ADHESION INHIBITORS**

BETA-ALANIN-DERIVATE ALS ZELL-ADHÄSIONS-INHIBITOREN

DERIVES D'ALANINE-BETA AGISSANT EN TANT QU'INHIBITEURS DE L'ADHESION CELLULAIRE

(84) Designated Contracting States:
**AT BE CH CY DE DK ES FI FR GB GR IE IT LI LU NL PT SE**
Designated Extension States:
**AL LT LV MK RO SI**

(30) Priority: **24.11.1997 US 66484 P**
**23.12.1997 GB 9727215**

(43) Date of publication of application:
**13.09.2000 Bulletin 2000/37**

(73) Proprietor: **MERCK & CO., INC.**
**Rahway, New Jersey 07065 (US)**

(72) Inventors:
- **DURETTE, Philippe, L.**
  **Rahway, NJ 07065 (US)**
- **HAGMANN, William, K.**
  **Rahway, NJ 07065 (US)**
- **KOPKA, Ihor, E.**
  **Rahway, NJ 07065 (US)**
- **MACCOSS, Malcolm**
  **Rahway, NJ 07065 (US)**
- **MILLS, Sander, G.**
  **Rahway, NJ 07065 (US)**
- **MUMFORD, Richard, A.**
  **Rahway, NJ 07065 (US)**
- **MAGRIOTIS, Plato, A.**
  **Rahway, NJ 07065 (US)**

(74) Representative: **Hiscock, Ian James et al**
**European Patent Department,**
**Merck & Co., Inc.,**
**Terlings Park,**
**Eastwick Road**
**Harlow, Essex CM20 2QR (GB)**

(56) References cited:

| | |
|---|---|
| **EP-A- 0 343 911** | **EP-A- 0 618 223** |
| **WO-A-95/35308** | **WO-A-96/22966** |
| **WO-A-97/20856** | **WO-A-98/47523** |
| **WO-A-98/53814** | **WO-A-99/06432** |
| **WO-A-99/06437** | **WO-A-99/61421** |
| **WO-A1-96/22966** | **WO-A1-97/03094** |
| **WO-A1-98/04913** | **DE-A- 4 445 500** |
| **GB-A- 2 292 149** | **US-A- 5 439 930** |

- **DATABASE CAOLD [Online] CHEMICAL ABSTRACTS SERVICE, COLUMBUS, OHIO, US; SMITH, EMIL L. ET AL: "enzymic and chem. properties of cryst. papain" retrieved from STN Database accession no. 2119b XP002158550**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- DATABASE CAOLD [Online] CHEMICAL ABSTRACTS SERVICE, COLUMBUS, OHIO, US; HALPERN, BERTHOLD ET AL: "high-sensitivity optical resolution of polyfunctional amino acids by gas-liquid chromatography" retrieved from STN Database accession no. 5524f XP002158551
- DATABASE CHEMABS [Online] CHEMICAL ABSTRACTS SERVICE, COLUMBUS, OHIO, US; NAGANO, MASANOBU ET AL: "Preparation of urea moiety-containing peptide derivatives as neutral endopeptidase and angiotensin converting enzyme inhibitors" retrieved from STN Database accession no. 127:66223 XP002158552 & JP 09 118662 A (FUJISAWA PHARMACEUTICAL CO., LTD., JAPAN) 6 May 1997 (1997-05-06) -& PATENT ABSTRACTS OF JAPAN vol. 1997, no. 09, 30 September 1997 (1997-09-30) & JP 09 118662 A , 6 May 1997 (1997-05-06)
- DATABASE CHEMABS [Online] CHEMICAL ABSTRACTS SERVICE, COLUMBUS, OHIO, US; TAKASUGI, HISASHI ET AL: "Amidino compounds as glycoprotein IIb/IIIa antagonists and pharmaceutical compositions containing them" retrieved from STN Database accession no. 123:285545 XP002158553 & JP 07 138221 A (FUJISAWA PHARMACEUTICAL CO, JAPAN) 30 May 1995 (1995-05-30)
- DATABASE CHEMABS [Online] CHEMICAL ABSTRACTS SERVICE, COLUMBUS, OHIO, US; YAMAMOTO, OSAMU ET AL: "Preparation of peptides as HCV protease inhibitors" retrieved from STN Database accession no. 131:310838 XP002166775 & JP 11 292840 A (SOYAKU GIJUTSU KENKYUSHO K. K., JAPAN) 26 October 1999 (1999-10-26)
- FERNANDEZ-MEGIA, EDUARDO ET AL: "On the Stereoselectivity of the Reaction of N-(9-Phenylfluoren-9- yl)aspartate Enolates with Electrophiles. Synthesis of Enantiomerically Pure 3-Hydroxy-, 3-Amino-, and 3-Hydroxy-3-methylaspartates" J. ORG. CHEM. (1994), 59(25), 7643-52, XP002166774
- DATABASE CHEMABS [Online] CHEMICAL ABSTRACTS SERVICE, COLUMBUS, OHIO, US; RESHETOVA, O. S. ET AL: "Analysis of N-dansyl peptide methyl esters by means high performance liquid chromatography and mass spectrometry" retrieved from STN Database accession no. 106:210288 XP002166776 & BIOORG. KHIM. (1987), 13(3), 320-37,
- CHEMICAL ABSTRACTS, Vol. 126, No. 15, 14 April 1997, (Columbus, Ohio, USA), page 603, Abstract No. 199840x, LIN K., "Preparation of Peptide Derivatives as Cell Adhesion Inhibitors", XP002916669; & WO 9703094 A, (30-01-97).
- CHEMICAL ABSTRACTS, Vol. 125, No. 19, 04 November 1996, (Columbus, Ohio, USA), page 1224, Abstract No. 248489e, ADAMS S., "Preparation of Dipeptide Derivatives as Cell Adhesion Inhibitors", XP002916670; & WO 9622966 A, (01-08-96).

## Description

BACKGROUND OF THE INVENTION

The present invention relates to novel substituted β-alanine derivatives which are useful for the inhibition and prevention of leukocyte adhesion and leukocyte adhesion-mediated pathologies. This invention also relates to compositions containing such compounds and methods of treatment using such compounds.

Many physiological processes require that cells come into close contact with other cells and/or extracellular matrix. Such adhesion events may be required for cell activation, migration, proliferation and differentiation. Cell-cell and cell-matrix interactions are mediated through several families of cell adhesion molecules (CAMs) including the selectins, integrins, cadherins and immunoglobulins. CAMs play an essential role in both normal and pathophysiological processes. Therefore, the targetting of specific and relevant CAMs in certain disease conditions without interfering with normal cellular functions is essential for an effective and safe therapeutic agent that inhibits cell-cell and cell-matrix interactions.

The integrin superfamily is made up of structurally and functionally related glycoproteins consisting of α and β heterodimeric, transmembrane receptor molecules found in various combinations on nearly every mammalian cell type. (for reviews see: E. C. Butcher, Cell, 67, 1033 (1991); T. A. Springer, Cell, 76, 301 (1994); D. Cox et al., "The Pharmacology of the Integrins." Medicinal Research Rev. 14, 195 (1994) and V. W. Engleman et al., "Cell Adhesion Integrins as Pharmaceutical Targets." in Ann. Repts. in Medicinal Chemistry, Vol. 31, J. A. Bristol, Ed.; Acad. Press, NY, 1996, p. 191).

VLA-4 ("very late antigen-4"; CD49d/CD29; or $\alpha_4\beta_1$) is an integrin expressed on all leukocytes, except platelets and mature neutrophils, including dendritic cells and macrophage-like cells and is a key mediator of the cell-cell and cell-matrix interactions of of these cell types (see M. E. Hemler, "VLA Proteins in the Integrin Family: Structures, Functions, and Their Role on Leukocytes." Ann. Rev. Immunol. 8, 365 (1990)). The ligands for VLA-4 include vascular cell adhesion molecule-1 (VCAM-1) and the CS-1 domain of fibronectin (FN). VCAM-1 is a member of the Ig superfamily and is expressed *in vivo* on endothelial cells at sites of inflammation. (See R. Lobb et al. "Vascular Cell Adhesion Molecule 1." in Cellular and Molecular Mechanisms of Inflammation, C. G. Cochrane and M. A. Gimbrone, Eds.; Acad. Press, San Diego, 1993, p. 151.) VCAM-1 is produced by vascular endothelial cells in response to pro-inflammatory cytokines (See A. J. H. Gearing and W. Newman, "Circulating adhesion molecules in disease.", Immunol. Today, 14, 506 (1993). The CS-1 domain is a 25 amino acid sequence that arises by alternative splicing within a region of fibronectin. (For a review, see R. O. Hynes "Fibronectins.", Springer-Velag, NY, 1990.) A role for VLA-4/CS-1 interactions in inflammatory conditions has been proposed (see M. J. Elices, "The integrin $\alpha_4\beta_1$ (VLA-4) as a therapeutic target" in Cell Adhesion and Human Disease, Ciba Found. Symp., John Wiley & Sons, NY, 1995, p. 79).

$\alpha_4\beta_7$ (also referred to as LPAM-1 and $\alpha_4\beta_p$) is an integrin expressed on leukocytes and is a key mediator of leukocyte trafficking and homing in the gastrointestinal tract (see C. M. Parker et al., Proc. Natl. Acad. Sci. USA, 89, 1924 (1992)). The ligands for $\alpha_4\beta_7$ include mucosal addressing cell adhesion molecule-1 (MadCAM-1) and, upon activation of $\alpha_4\beta_7$, VCAM-1 and fibronectin (Fn). MadCAM-1 is a member of the Ig superfamily and is expressed in vivo on endothelial cells of gut-associated mucosal tissues of the small and large intestine ("Peyer's Patches") and lactating mammary glands. (See M. J. Briskin et al., Nature, 363, 461 (1993); A. Hamann et al., J. Immunol., 152, 3282 (1994)). MadCAM-1 can be induced in vitro by proinflammatory stimuli (See E. E. Sikorski et al. J. Immunol., 151, 5239 (1993)). MadCAM-1 is selectively expressed at sites of lymphocyte extravasation and specifically binds to the integrin, $\alpha_4\beta_7$.

Neutralizing anti-$\alpha_4$ antibodies or blocking peptides that inhibit the interaction between VLA-4 and/or $\alpha_4\beta_7$ and their ligands have proven efficacious both prophylactically and therapeutically in several animal models of disease, including i) experimental allergic encephalomyelitis, a model of neuronal demyelination resembling multiple sclerosis (for example, see T. Yednock et al., "Prevention of experimental autoimmune encephalomyelitis by antibodies against $\alpha_4\beta_1$ integrin." Nature, 356, 63 (1993) and E. Keszthelyi et al., "Evidence for a prolonged role of $\alpha_4$ integrin throughout active experimental allergic encephalomyelitis." Neurology, 47, 1053 (1996)); ii) bronchial hyperresponsiveness in sheep and guinea pigs as models for the various phases of asthma (for example, see W. M. Abraham et al., "$\alpha_4$-Integrins mediate antigen-induced late bronchial responses and prolonged airway hyperresponsiveness in sheep." J. Clin. Invest. 93, 776 (1993) and A. A. Y. Milne and P. P. Piper, "Role of VLA-4 integrin in leucocyte recruitment and bronchial hyperresponsiveness in the gunea-pig." Eur. J. Pharmacol., 282, 243 (1995)); iii) adjuvant-induced arthritis in rats as a model of inflammatory arthritis (see C. Barbadillo et al., "Anti-VLA-4 mAb prevents adjuvant arthritis in Lewis rats." Arthr. Rheuma. (Suppl.), 36 95 (1993) and D. Seiffge, "Protective effects of monoclonal antibody to VLA-4 on leukocyte adhesion and course of disease in adjuvant arthritis in rats." J. Rheumatol., 23, 12 (1996)); iv) adoptive autoimmune diabetes in the NOD mouse (see J. L. Baron et al., "The pathogenesis of adoptive murine autoimmune diabetes requires an interaction between $\alpha_4$-integrins and vascular cell adhesion molecule-1.", J. Clin. Invest., 93, 1700 (1994), A. Jakubowski et al., "Vascular cell adhesion molecule-Ig fusion protein selectively targets activated α4-integrin receptors in vivo: Inhibition of autoimmune diabetes in an adoptive transfer model in nonobese diabetic mice." J. Immunol., 155, 938 (1995), and X. D. Yang et al., "Involvement of beta 7 integrin and mucosal addressin cell adhesion molecule-1 (MadCAM-1) in the development of diabetes in nonobese diabetic mice", Diabetes, 46, 1542 (1997)); v) cardiac allograft survival in mice as a model of organ transplantation (see M. Isobe et al., "Effect of anti-VCAM-1 and anti-VLA-4 monoclonal antibodies on cardiac allograft survival and response to soluble antigens in mice.", Tranplant. Proc., 26, 867 (1994) and S. Molossi et al., "Blockade of very late antigen-4 integrin binding to fibronectin with connecting segment-1 peptide reduces accelerated coronary arteripathy in rabbit cardiac allografts." J. Clin Invest., 95, 2601 (1995)); vi) spontaneous chronic colitis in cotton-top tamarins which resembles human ulcerative colitis, a form of inflammatory bowel disease (see D. K. Podolsky et al., "Attenuation of colitis in the Cotton-top tamarin by anti-$\alpha_4$ integrin monoclonal antibody.", J. Clin. Invest., 92, 372 (1993)); vii) contact hypersensitivity models as a model for skin allergic reactions (see T. A. Ferguson and T. S. Kupper, "Antigen-independent processes in antigen-specific immunity.", J. Immunol., 150, 1172 (1993) and P. L. Chisholm et al., "Monoclonal antibodies to the integrin $\alpha$-4 subunit inhibit the murine contact hypersensitivity response." Eur. J. Immunol., 23, 682 (1993)); viii) acute neurotoxic nephritis (see M. S. Mulligan et al., "Requirements for leukocyte adhesion molecules in nephrotoxic nephritis.", J. Clin. Invest., 91, 577 (1993)); ix) tumor metastasis (for examples, see M. Edward, "Integrins and other adhesion molecules involved in melanocytic tumor progression.", Curr. Opin. Oncol., 7, 185 (1995)); x) experimental autoimmune thyroiditis (see R. W. McMurray et al., "The role of $\alpha$4 integrin and intercellular adhesion molecule-1 (ICAM-1) in murine experimental autoimmune thyroiditis." Autoimmunity, 23, 9 (1996); and xi) ischemic tissue damage following arterial occlusion in rats (see F. Squadrito et al., "Leukocyte integrin very late antigen-4/vascular cell adhesion molecule-1 adhesion pathway in splanchnic artery occlusion shock." Eur. J. Pharmacol., 318, 153 (1996; xii) inhibition of TH2 T-cell cytokine production including IL-4 and IL-5 by VLA-4 antibodies which would attenuate allergic responses (J.Clinical Investigation 100, 3083 (1997). The primary mechanism of action of such antibodies appears to be the inhibition of lymphocyte and monocyte interactions with CAMs associated with components of the extracellular matrix, thereby limiting leukocyte migration to extravascular sites of injury or inflammation and/or limiting the priming and/or activation of leukocytes.

There is additional evidence supporting a possible role for VLA-4 interactions in other diseases, including rheumatoid arthritis; various melanomas, carcinomas, and sarcomas; inflammatory lung disorders; acute respiratory distress syndrome (ARDS); atherosclerotic plaque formation; restenosis; uveitis and circulatory shock (for examples, see A. A. Postigo et al., "The $\alpha_4\beta_1$/VCAM-1 adhesion pathway in physiology and disease.", Res. Immunol., 144, 723 (1994) and J.-X. Gao and A. C. Issekutz, "Expression of VCAM-1 and VLA-4 dependent T-lymphocyte adhesion to dermal fibroblasts stimulated with proinflammatory cytokines." Immunol. 89, 375 (1996)).

At present, there is a humanized monoclonal antibody (Antegren® Athena Neurosciences/Elan ) against VLA-4 in clinical development for the treatment of "flares" associated with multiple sclerosis and a humanized monoclonal antibody (ACT-1®/LDP-02 LeukoSite) against $\alpha_4\beta_7$ in clinical development for the treatment of inflammatory bowel disease. Several peptidyl antagonists of VLA-4 have been described (D. Y. Jackson et al., "Potent $\alpha4\beta1$ peptide antagonists as potential anti-inflammatory agents", J. Med. Chem., 40, 3359 (1997); H. N. Shroff et al., "Small peptide inhibitors of $\alpha4\beta7$ mediated MadCAM-1 adhesion to lymphocytes", Bioorg. Med. Chem. Lett., 6, 2495 (1996); US 5,510,332, WO97/03094, WO97/02289, WO96/40781, WO96/22966, WO96/20216, WO96/01644, WO96/06108, WO95/15973). There is one report of nonpeptidyl inhibitors of the ligands for $\alpha_4$-integrins (WO96/31206). There still remains a need for low molecular weight, specific inhibitors of VLA-4- and $\alpha4\beta7$-dependent cell adhesion that have improved pharmacokinetic and pharmacodynamic properties such as oral bioavailability and significant duration of action. Such compounds would prove to be useful for the treatment, prevention or suppression of various pathologies mediated by VLA-4 and $\alpha4\beta7$ binding and cell adhesion and activation.

## SUMMARY OF THE INVENTION

The compounds of the present invention are antagonists of the VLA-4 integrin ("very late antigen-4"; CD49d/CD29; or $\alpha_4\beta_1$) and/or the $\alpha4\beta7$ integrin (LPAM-1 and $\alpha_4\beta_p$), thereby blocking the binding of VLA-4 to its various ligands, such as VCAM-1 and regions of fibronectin and/or $\alpha4\beta7$ to its various ligands, such as MadCAM-1, VCAM-1 and fibronectin. Thus, these antagonists are useful in inhibiting cell adhesion processes including cell activation, migration, proliferation and differentiation. These antagonists are useful in the treatment, prevention and suppression of diseases mediated by VLA-4 and/or $\alpha4\beta7$ binding and cell adhesion and activation, such as multiple sclerosis, asthma, allergic rhinitis, allergic conjunctivitis, inflammatory lung diseases, rheumatoid arthritis, septic arthritis, type I diabetes, organ transplantation, restenosis, autologous bone marrow transplantation, inflammatory sequelae of viral infections, myocarditis, inflammatory bowel disease including ulcerative colitis and Crohn's disease, certain types of toxic and immune-based nephritis, contact dermal hypersensitivity, psoriasis, tumor metastasis, and atherosclerosis.

## DETAILED DESCRIPTION OF THE INVENTION

The present invention provides novel compounds of Formula I

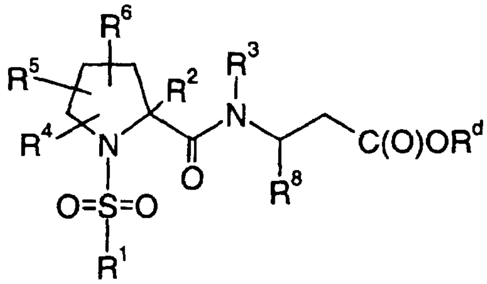

I

or a pharmaceutically acceptable salt thereof wherein:

$R^1$ is phenyl optionally substituted with one to four substituents independently selected from $R^b$;
$R^2$ is hydrogen or $C_{1-6}$alkyl;
$R^3$ is

1) hydrogen,
2) $C_{1-10}$ alkyl,
3) Cy, or
4) Cy-$C_{1-10}$ alkyl,

wherein alkyl is optionally substituted with one to four substituents independently selected from $R^a$; and Cy is optionally substituted with one to four substituents independently selected from $R^b$;
$R^4$, $R^5$ and $R^6$ are each independently selected from the group consisting of

1) hydrogen,or
2) a group selected from $R^b$;

$R^8$ is Cy-($Cy^1$) wherein Cy and $Cy^1$ are each phenyl optionally substituted with one to four substituents independently selected from $R^b$;
$R^a$ is

1) $-CF_3$,
2) $-OR^d$,
3) $-NO_2$,
4) halogen
5) $-S(O)_mR^d$,
6) $-SR^d$,
7) $-S(O)_2OR^d$,
8) $-S(O)_mNR^dR^e$,
9) $-NR^dR^e$,
10) $-O(CR^fR^g)_nNR^dR^e$,
11) $-C(O)R^d$,
12) $-CO_2R^d$,
13) $-CO_2(CR^fR^g)_nCONR^dR^e$,
14) $-OC(O)R^d$,
15) -CN,
16) $-C(O)NR^dR^e$,
17) $-NR^dC(O)R^e$,
18) -OC(O)NRdRe,
19) $-NR^dC(O)OR^e$,
20) $-NR^dC(O)Nr^dR^e$,
21) $-CR^d(N-OR^e)$, or
22) Cy optionally substituted with a group independently selected from $R^c$;

$R^b$ is

1) a group selected from $R^a$,
2) $C_{1-10}$ alkyl,
3) $C_{2-10}$ alkenyl,
4) $C_{2-10}$ alkynyl, or
5) Cy-$C_{1-10}$ alkyl,

wherein alkyl, alkenyl, alkynyl, and Cy are optionally substituted with a group independently selected from $R^c$;
$R^c$ is

1) halogen,
2) CN,
3) NH($C_{1-5}$alkyl),
4) N($C_{1-5}$alkyl)$_2$,
5) amino,
6) carboxy,
7) $C_{1-4}$alkyl,
8) $C_{1-4}$alkoxy,
9) aryl,
10) aryl $C_{1-4}$alkyl, or
11) aryloxy;

$R^d$ and $R^e$ are independently selected from hydrogen, $C_{1-10}$alkyl, $C_{2-10}$alkenyl, $C_{2-10}$alkynyl, Cy and Cy-$C_{1-10}$alkyl, wherein alkyl, alkenyl, alkynyl and Cy is optionally substituted with one to four substituents independently selected from $R^c$; or
$R^d$ and $R^e$ together with the atoms to which they are attached form a heterocyclic ring of 5 to 7 members containing 0-2 additional heteroatoms independently selected from oxygen, sulfur and nitrogen;
$R^f$ and $R^g$ are independently selected from hydrogen, $C_{1-10}$alkyl, Cy and Cy-$C_{1-10}$alkyl; or
$R^f$ and $R^g$ together with the carbon to which they are attached form a ring of 5 to 7 members containing 0-2 heteroatoms independently selected from oxygen, sulfur and nitrogen;
Cy and $Cy^1$are

1) cycloalkyl,
2) heterocyclyl,
3) aryl, or
4) heteroaryl;

m is an integer from 1 to 2; and
n is an integer from 1 to 10.

In one subset of compounds of formula I $R^1$ is phenyl optionally substituted with one or two groups independently selected from halogen, O-$C_{1-3}$alkyl, and trifluoromethyl. A more preferred $R^1$ is 3,5-dichlorophenyl (3,5-diCl-Ph) or (3-$CF_3$-Ph).
In another subset of compounds of formula I $R^2$ is H or methyl.
In another subset of compounds of formula I $R^8$ is optionally substituted biphenyl, wherein the optional substituents are one or two groups independently selected from halogen, CN, $OR^d$, O(CO)$R^d$, $C_{1-5}$alkyl optionally substituted with one or two groups selected from $R^c$, $CF_3$, and OC(O)$NR^dR^e$; $R^c$, $R^d$ and $R^e$ are as defined under formula I. Examples of preferred R8 include biphenyl, 2'-methoxybiphenyl, 2'-cyanobiphenyl, 2'-formylbiphenyl, 2'-dimethylaminomethylbiphenyl, 2'-hydroxy-methylbiphenyl, 4'-fluorobiphenyl, 2'-$CF_3$O-biphenyl, 3'-methoxybiphenyl, 2'-methoxy-3'-fluorobiphenyl, 3'-methoxy-2'-fluorobiphenyl, 2'-methoxy-5'-fluoro-biphenyl, 3'-methoxy-5'-fluoro-biphenyl, 2'-methoxy-6'-fluorobiphenyl, 2'-$CF_3$O-4'-fluorobiphenyl, 2'-methoxy-4'-fluorobiphenyl, 3-(2'-methoxyphenyl)phenyl, 4-(2'-cyclopropoxy)biphenyl.
A preferred embodiment of compounds of formula I are compounds of formula Ib:

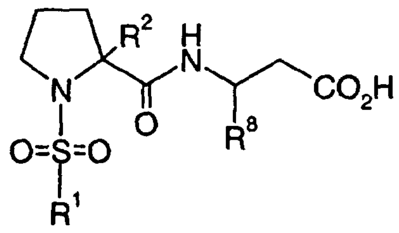

Ib

wherein
$R^1$ is phenyl optionally substituted as provided above under formula I;
$R^2$ is H or $C_{1-6}$ alkyl; and
$R^8$ is Cy-($Cy^1$), wherein Cy and $Cy^1$ are each phenyl optionally substituted as provided above under formula I.

In a more preferred embodiment of compounds of formula Ib, $R^1$ is phenyl optionally substituted with one or two groups selected from halogen, O-$C_{1-3}$alkyl, and trifluoromethyl ;
$R^2$ is H or methyl;
$R^8$ is optionally substituted biphenyl wherein the optional substituents are one or two groups independently selected from halogen, CN, $OR^d$, $O(CO)R^d$, $C_{1-5}$alkyl optionally substituted with one or two groups selected from $R^c$, $CF_3$, and $OC(O)NR^dR^e$; $R^c$, $R^d$ and $R^e$ are as defined under formula I.

Representative compounds of formula I are as follows (biphenyl is 4-biphenyl, unless otherwise specified):

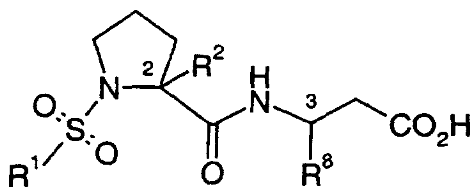

| 2/3* | $R^1$ | $R^2$ | $R^8$ |
|---|---|---|---|
| S/R | 3,5-diCl-Ph | H | biphenyl |
| S/S | 3,5-diCl-Ph | H | biphenyl |
| S/R | 3,5-diCl-Ph | H | 2'-methoxybiphenyl |
| S/S | 3,5-diCl-Ph | H | 2'-methoxybiphenyl |
| S/R | 3,5-diCl-Ph | H | 2'-cyanobiphenyl |
| S/R | 3,5-diCl-Ph | H | 2'-formylbiphenyl |
| S/R | 3,5-diCl-Ph | H | 2'-dimethylaminomethylbiphenyl |
| S/R | 3,5-diCl-Ph | H | 2'-hydroxymethylbiphenyl |
| S/R | Ph | H | 2'-methoxybiphenyl |
| S/R | Ph | $CH_3$ | 2'-methoxybiphenyl |
| S/R | 3,5-diCl-Ph | $CH_3$ | 2'-methoxybiphenyl |
| S/R | Ph | $CH_3$ | 4'-fluorobiphenyl |
| S/R | 3,5-diCl-Ph | H | 4'-fluorobiphenyl |
| S/R | 3,5-diCl-Ph | H | 2'-$CF_3$O-biphenyl |
| S/R | 3,5-diCl-Ph | $CH_3$ | 2'-$CF_3$O-biphenyl |
| S/R | 3,5-diCl-Ph | $CH_3$ | 3'-methoxybiphenyl |
| S/R | 3,5-diCl-Ph | H | 3'-methoxybiphenyl |

*Stereoconfiguration at the indicated positions

(continued)

| 2/3* | $R^1$ | $R^2$ | $R^8$ |
|---|---|---|---|
| S/R | 3,5-diCl-Ph | $CH_3$ | 2'-methoxy-3'-F-biphenyl |
| S/R | 3,5-diCl-Ph | H | 2'-methoxy-3'-F-biphenyl |
| S/R | 3,5-diCl-Ph | $CH_3$ | 3'-methoxy-2'-F-biphenyl |
| S/R | 3,5-diCl-Ph | H | 3'-methoxy-2'-F-biphenyl |
| S/R | 3,5-diCl-Ph | $CH_3$ | 2'-methoxy-5'-F-biphenyl |
| S/R | 3,5-diCl-Ph | H | 2'-methoxy-5'-F-biphenyl |
| S/R | 3,5-diCl-Ph | $CH_3$ | 3'-methoxy-5'-F-biphenyl |
| S/R | 3,5-diCl-Ph | H | 3'-methoxy-5'-F-biphenyl |
| S/R | 3,5-diCl-Ph | $CH_3$ | 2'-methoxy-6'-F-biphenyl |
| S/R | 3,5-diCl-Ph | H | 2'-methoxy-6'-F-biphenyl |
| S/R | 3-Cl-Ph | $CH_3$ | 2'-methoxybiphenyl |
| S/R | 3,5-diCl-Ph | $CH_3$ | 2'-$CF_3$O-4'-F-biphenyl |
| S/R | 3,5-diCl-Ph | H | 2'-$CF_3$O-4'-F-biphenyl |
| S/R | 3,5-diCl-Ph | H | 2'-methoxy-4'-F-biphenyl |
| S/R | 3,5-diCl-Ph | $CH_3$ | 2'-methoxy-4'-F-biphenyl |
| S/R | 3,5-diCl-Ph | H | 3-(2'-methoxyphenyl)phenyl |
| S/R | 3,5-diCl-Ph | H | 4-(2'-cyclopropoxy)biphenyl |
| S/R | 3,5-diCl-Ph | $CH_3$ | 4-(2'-cyclopropoxy)biphenyl |

*Stereoconfiguration at the indicated positions

"Alkyl", as well as other groups having the prefix "alk", such as alkoxy, alkanoyl, means carbon chains which may be linear or branched or combinations thereof. Examples of alkyl groups include methyl, ethyl, propyl, isopropyl, butyl, sec- and tert-butyl, pentyl, hexyl, heptyl, octyl, nonyl, and the like.

"Alkenyl" means carbon chains which contain at least one carbon-carbon double bond, and which may be linear or branched or combinations thereof. Examples of alkenyl include vinyl, allyl, isopropenyl, pentenyl, hexenyl, heptenyl, 1-propenyl, 2-butenyl, 2-methyl-2-butenyl, and the like.

"Alkynyl" means carbon chains which contain at least one carbon-carbon triple bond, and which may be linear or branched or combinations thereof. Examples of alkynyl include ethynyl, propargyl, 3-methyl-1-pentynyl, 2-heptynyl and the like.

"Cycloalkyl" means mono- or bicyclic saturated carbocyclic rings, each of which having from 3 to 10 carbon atoms. The term also inccludes monocyclic ring fused to an aryl group in which the point of attachment is on the non-aromatic portion. Examples of cycloalkyl include cyclopropyl, cyclopentyl, cyclohexyl, cycloheptyl, tetrahydronaphthyl, decahydronaphthyl, indanyl, and the like.

"Aryl" means mono- or bicyclic aromatic rings containing only carbon atoms. The term also includes aryl group fused to a monocyclic cycloalkyl or monocyclic heterocyclyl group in which the point of attachment is on the aromatic portion. Examples of aryl include phenyl, naphthyl, indanyl, indenyl, tetrahydronaphthyl, 2,3-dihydrobenzofuranyl, benzopyranyl, 1,4-benzodioxanyl, 1,3-benzodioxolyl, and the like.

"Heteroaryl" means a mono- or bicyclic aromatic ring containing at least one heteroatom selected from N, O and S, with each ring containing 5 to 6 atoms. Examples of heteroaryl include pyrrolyl, isoxazolyl, isothiazolyl, pyrazolyl, pyridyl, oxazolyl, oxadiazolyl, thiadiazolyl, thiazolyl, imidazolyl, triazolyl, tetrazolyl, furanyl, triazinyl, thienyl, pyrimidyl, pyridazinyl, pyrazinyl, benzoxazolyl, benzothiazolyl, benzimidazolyl, benzofuranyl, benzothiophenyl, furo(2,3-b)pyridyl, quinolyl, indolyl, isoquinolyl, and the like.

"Heterocyclyl" means mono- or bicyclic saturated rings containing at least one heteroatom selected from N, S and O, each of said ring having from 3 to 10 atoms. The term also includes monocyclic heterocycle fused to an aryl or heteroaryl group in which the point of attachment is on the non-aromatic portion. Examples of "heterocyclyl" include pyrrolidinyl, piperidinyl, piperazinyl, imidazolidinyl, 2,3-dihydrofuro(2,3-b)pyridyl, benzoxazinyl, tetrahydroquinolinyl, tetrahydroisoquinolinyl, dihydroindolyl, and the like. The term also includes partially unsaturated monocyclic rings that are not aromatic, such as 2- or 4-pyridones or N-substituted-(1H,3H)-pyrimidine-2,4-diones (N-substituted uracils).

"Halogen" includes fluorine, chlorine, bromine and iodine.

Some of the following abbreviations are used in the application:

BOC (boc) t-butyloxycarbonyl

| | |
|---|---|
| Bu | butyl |
| calc. | calculated |
| CBZ (Cbz) | benzyloxycarbonyl |
| DCC | dicyclohexylcarbodiimide |
| DIEA | diisopropylethylamine |
| DMAP | 4-(N,N-dimethylamino)pyridine |
| DMF | dimethylformamide |
| DMSO | dimethylsulfoxide |
| EDC | 1-(3-dimethylaminopropyl)3-ethylcarbodiimide HCl |
| eq. | equivalent(s) |
| EtOAc | ethyl acetate |
| FAB-MS | fast atom bombardment-mass spectroscopy |
| FMOC (Fmoc) | fluororenylmethoxycarbonyl |
| HBTU | 2-(1H-benzotriazol-1-yl)-1,1,3,3-tetramethyluronium hexafluorophosphate |
| HOBt | 1-hydroxybenzotriazole hydrate |
| HPLC | high pressure liquid chromatography |
| K/Li HDMS | potassium/lithium bis(trimethylsilyl)amide |
| LAH | lithium aluminum hydride |
| LHMDS | lithium bis(trimethylsilyl)amide |
| Me | methyl |
| MF | molecular formula |
| MHz | megahertz |
| Ms | methanesulfonyl |
| NBS | N-bromosuccinimde |
| NMM | N-methylmorpholine |
| NMP | N-methylpyrrolidin-2-one |
| NMR | nuclear magnetic resonance |
| Ph | phenyl |
| Pr | propyl |
| prep. | prepared |
| PyBOP | benzotriazol-1-yloxytripyrrolidino phosphonium hexafluorophosphate |
| TFA | trifluoroacetic acid |
| THF | tetrahydrofuran |
| Tic | 1,2,3,4-tetrahydroisoquinoline-3-carboxylic acid |
| TLC | thin-layer chromatography |

Optical Isomers - Diastereomers - Geometric Isomers - Tautomers

Compounds of Formula I contain one or more asymmetric centers and can thus occur as racemates and racemic mixtures, single enantiomers, diastereomeric mixtures and individual diastereomers. The present invention is meant to comprehend all such isomeric forms of the compounds of Formula I.

Some of the compounds described herein contain olefinic double bonds, and unless specified otherwise, are meant to include both E and Z geometric isomers.

Some of the compounds described herein may exist with different points of attachment of hydrogen, referred to as tautomers. Such an example may be a ketone and its enol form known as keto-enol tautomers. The individual tautomers as well as mixture thereof are encompassed with compounds of Formula I.

Compounds of the Formula I may be separated into diastereoisomeric pairs of enantiomers by, for example, fractional crystallization from a suitable solvent, for example methanol or ethyl acetate or a mixture thereof. The pair of enantiomers thus obtained may be separated into individual stereoisomers by conventional means, for example by the use of an optically active acid as a resolving agent.

Alternatively, any enantiomer of a compound of the general Formula I or Ia may be obtained by stereospecific synthesis using optically pure starting materials or reagents of known configuration.

Salts

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous,

potassium, sodium, zinc, and the like. Particularly preferred are the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-dibenzylethylenediamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid, and the like. Particularly preferred are citric, hydrobromic, hydrochloric, maleic, phosphoric, sulfuric, and tartaric acids.

It will be understood that, as used herein, references to the compounds of Formula I are meant to also include the pharmaceutically acceptable salts.

## Utilities

The ability of the compounds of Formula I to antagonize the actions of VLA-4 and/or α4β7 integrin makes them useful for preventing or reversing the symptoms, disorders or diseases induced by the binding of VLA-4 and or α4β7to their various respective ligands. Thus, these antagonists will inhibit cell adhesion processes including cell activation, migration, proliferation and differentiation. Accordingly, another aspect of the present invention provides a method for the treatment (including prevention, alleviation, amelioration or suppression) of diseases or disorders or symptoms mediated by VLA-4 and/or α4β7 binding and cell adhesion and activation, which comprises administering to a mammal an effective amount of a compound of Formula I. Such diseases, disorders, conditions or symptoms are for example (1) multiple sclerosis, (2) asthma, (3) allergic rhinitis, (4) allergic conjunctivitis, (5) inflammatory lung diseases, (6) rheumatoid arthritis, (7) septic arthritis, (8) type I diabetes, (9) organ transplantation rejection, (10) restenosis, (11) autologous bone marrow transplantation, (12) inflammatory sequelae of viral infections, (13) myocarditis, (14) inflammatory bowel disease including ulcerative colitis and Crohn's disease, (15) certain types of toxic and immune-based nephritis, (16) contact dermal hypersensitivity, (17) psoriasis, (18) tumor metastasis, (19) hepatitis, and (20) atherosclerosis.

## Dose Ranges

The magnitude of prophylactic or therapeutic dose of a compound of Formula I will, of course, vary with the nature of the severity of the condition to be treated and with the particular compound of Formula I and its route of administration. It will also vary according to the age, weight and response of the individual patient. In general, the daily dose range lie within the range of from about 0.001 mg to about 100 mg per kg body weight of a mammal, preferably 0.01 mg to about 50 mg per kg, and most preferably 0.1 to 10 mg per kg, in single or divided doses. On the other hand, it may be necessary to use dosages outside these limits in some cases.

For use where a composition for intravenous administration is employed, a suitable dosage range is from about 0.001 mg to about 25 mg (preferably from 0.01 mg to about 1 mg) of a compound of Formula I per kg of body weight per day and for cytoprotective use from about 0.1 mg to about 100 mg (preferably from about 1 mg to about 100 mg and more preferably from about 1 mg to about 10 mg) of a compound of Formula I per kg of body weight per day.

In the case where an oral composition is employed, a suitable dosage range is, e.g. from about 0.01 mg to about 100 mg of a compound of Formula I per kg of body weight per day, preferably from about 0.1 mg to about 10 mg per kg and for cytoprotective use from 0.1 mg to about 100 mg (preferably from about 1 mg to about 100 mg and more preferably from about 10 mg to about 100 mg) of a compound of Formula I per kg of body weight per day.

For the treatment of diseases of the eye, ophthalmic preparations for ocular administration comprising 0.001-1% by weight solutions or suspensions of the compounds of Formula I in an acceptable ophthalmic formulation may be used.

## Pharmaceutical Compositions

Another aspect of the present invention provides pharmaceutical compositions which comprises a compound of Formula I and a pharmaceutically acceptable carrier. The term "composition", as in pharmaceutical composition, is intended to encompass a product comprising the active ingredient(s), and the inert ingredient(s) (pharmaceutically

acceptable excipients) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of Formula I, additional active ingredient(s), and pharmaceutically acceptable excipients.

Any suitable route of administration may be employed for providing a mammal, especially a human with an effective dosage of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like.

The pharmaceutical compositions of the present invention comprise a compound of Formula I as an active ingredient or a pharmaceutically acceptable salt thereof, and may also contain a pharmaceutically acceptable carrier and optionally other therapeutic ingredients. The term "pharmaceutically acceptable salts" refers to salts prepared from pharmaceutically acceptable non-toxic bases or acids including inorganic bases or acids and organic bases or acids.

The compositions include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

For administration by inhalation, the compounds of the present invention are conveniently delivered in the form of an aerosol spray presentation from pressurized packs or nebulisers. The compounds may also be delivered as powders which may be formulated and the powder composition may be inhaled with the aid of an insufflation powder inhaler device. The preferred delivery system for inhalation is a metered dose inhalation (MDI) aerosol, which may be formulated as a suspension or solution of a compound of Formula I in suitable propellants, such as fluorocarbons or hydrocarbons.

Suitable topical formulations of a compound of formula I include transdermal devices, aerosols, creams, ointments, lotions, dusting powders, and the like.

In practical use, the compounds of Formula I can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, capsules and tablets, with the solid oral preparations being preferred over the liquid preparations. Because of their ease of administration, tablets and capsules represent the most advantageous oral dosage unit form in which case solid pharmaceutical carriers are obviously employed. If desired, tablets may be coated by standard aqueous or nonaqueous techniques.

In addition to the common dosage forms set out above, the compounds of Formula I may also be administered by controlled release means and/or delivery devices such as those described in U.S. Patent Nos. 3,845,770; 3,916,899; 3,536,809; 3,598,123; 3,630,200 and 4,008,719.

Pharmaceutical compositions of the present invention suitable for oral administration may be presented as discrete units such as capsules, cachets or tablets each containing a predetermined amount of the active ingredient, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Desirably, each tablet contains from about 1 mg to about 500 mg of the active ingredient and each cachet or capsule contains from about 1 to about 500 mg of the active ingredient.

The following are examples of representative pharmaceutical dosage forms for the compounds of Formula I:

| Injectable Suspension (I.M.) | mg/mL |
|---|---|
| Compound of Formula I | 10 |
| Methylcellulose | 5.0 |
| Tween 80 | 0.5 |
| Benzyl alcohol | 9.0 |
| Benzalkonium chloride | 1.0 |
| Water for injection to a total volume of 1 mL | |

| Tablet | mg/tablet |
|---|---|
| Compound of Formula I | 25 |
| Microcrystalline Cellulose | 415 |
| Povidone | 14.0 |
| Pregelatinized Starch | 43.5 |
| Magnesium Stearate | 2.5 |
| | 500 |

| Capsule | mg/capsule |
|---|---|
| Compound of Formula I | 25 |
| Lactose Powder | 573.5 |
| Magnesium Stearate | 1.5 |
| | 600 |

| Aerosol | Per canister |
|---|---|
| Compound of Formula I | 24 mg |
| Lecithin, NF Liquid Concentrate | 1.2 mg |
| Trichlorofluoromethane, NF | 4.025 g |
| Dichlorodifluoromethane, NF | 12.15 g |

Combination Therapy

Compounds of Formula I may be used in combination with other drugs that are used in the treatment/prevention/suppression or amelioration of the diseases or conditions for which compounds of Formula I are useful. Such other drugs may be administered, by a route and in an amount commonly used therefor, contemporaneously or sequentially with a compound of Formula I. When a compound of Formula I is used contemporaneously with one or more other drugs, a pharmaceutical composition containing such other drugs in addition to the compound of Formula I is preferred. Accordingly, the pharmaceutical compositions of the present invention include those that also contain one or more other active ingredients, in addition to a compound of Formula I. Examples of other active ingredients that may be combined with a compound of Formula I, either administered separately or in the same pharmaceutical compositions, include, but are not limited to:

(a) other VLA-4 antagonists such as those described in US 5,510,332, WO97/03094, WO97/02289, WO96/40781, WO96/22966, WO96/20216, WO96/01644, WO96/06108, WO95/15973 and WO96/31206; (b) steroids such as beclomethasone, methylprednisolone, betamethasone, prednisone, dexamethasone, and hydrocortisone; (c) immunosuppressants such as cyclosporin, tacrolimus, rapamycin and other FK-506 type immunosuppressants; (d) antihistamines (H1-histamine antagonists) such as bromopheniramine, chlorpheniramine, dexchlorpheniramine, triprolidine, clemastine, diphenhydramine, diphenylpyraline, tripelennamine, hydroxyzine, methdilazine, promethazine, trimeprazine, azatadine, cyproheptadine, antazoline, pheniramine pyrilamine, astemizole, terfenadine, loratadine, cetirizine, fexofenadine, descarboethoxyloratadine, and the like; (e) non-steroidal anti-asthmatics such as β2-agonists (terbutaline, metaproterenol, fenoterol, isoetharine, albuterol, bitolterol, and pirbuterol), theophylline, cromolyn sodium, atropine, ipratropium bromide, leukotriene antagonists (zafirlukast, montelukast, pranlukast, iralukast, pobilukast, SKB-106,203), leukotriene biosynthesis inhibitors (zileuton, BAY-1005); (f) non-steroidal antiinflammatory agents (NSAIDs) such as propionic acid derivatives (alminoprofen, benoxaprofen, bucloxic acid, carprofen, fenbufen, fenoprofen, fluprofen, flurbiprofen, ibuprofen, indoprofen, ketoprofen, miroprofen, naproxen, oxaprozin, pirprofen, pranoprofen, suprofen, tiaprofenic acid, and tioxaprofen), acetic acid derivatives (indomethacin, acemetacin, alclofenac, clidanac, diclofenac, fenclofenac, fenclozic acid, fentiazac, furofenac, ibufenac, isoxepac, oxpinac, sulindac, tiopinac, tolmetin, zidometacin, and zomepirac), fenamic acid derivatives (flufenamic acid, meclofenamic acid, mefenamic acid, niflumic acid and tolfenamic acid), biphenylcarboxylic acid derivatives (diflunisal and flufenisal), oxicams (isoxicam, piroxicam, sudoxicam and tenoxican), salicylates (acetyl salicylic acid, sulfasalazine) and the pyrazolones (apazone, bezpiperylon, feprazone, mofebutazone, oxyphenbutazone, phenylbutazone); (g) cyclooxygenase-2 (COX-2) inhibitors such as celecoxib; (h) inhibitors of phosphodiesterase type IV (PDE-IV); (i) antagonists of the chemokine receptors, especially CCR-1, CCR-2, and CCR-3; (j) cholesterol lowering agents such as HMG-CoA reductase inhibitors (lovastatin, simvastatin and pravastatin, fluvastatin, atorvastatin, and other statins), sequestrants (cholestyramine and colestipol), nicotinic acid, fenofibric acid derivatives (gemfibrozil, clofibrat, fenofibrate and benzafibrate), and probucol; (k) anti-diabetic agents such as insulin, sulfonylureas, biguanides (metformin), α-glucosidase inhibitors (acarbose) and glitazones (troglitazone, pioglitazone, englitazone, MCC-555, BRL49653 and the like); (l) preparations of interferon beta (interferon beta-1a, interferon beta-1b); (m) anticholinergic agents such as muscarinic antagonists (ipratropium bromide); (n) other compounds such as 5-aminosalicylic acid and prodrugs thereof, antimetabolites such as azathioprine and 6-mercaptopurine, and cytotoxic cancer chemotherapeutic agents.

The weight ratio of the compound of the Formula I to the second active ingredient may be varied and will depend upon the effective dose of each ingredient. Generally, an effective dose of each will be used. Thus, for example, when a compound of the Formula I is combined with an NSAID the weight ratio of the compound of the Formula I to the NSAID will generally range from about 1000:1 to about 1:1000, preferably about 200:1 to about 1:200. Combinations of a compound of the Formula I and other active ingredients will generally also be within the aforementioned range, but in each case, an effective dose of each active ingredient should be used.

Compounds of the present invention may be prepared by procedures illustrated in the accompanying schemes. In Scheme 1, a resin-based synthetic strategy is outlined where the resin employed is represented by the ball (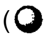). An N-Fmoc-protected amino acid derivative **A** (Fmoc = fluorenylmethoxycarbonyl) is loaded on to the appropriate hydroxyl-containing resin using a coupling agent such as dicyclohexylcarbodiimide (DCC) or 1-(3-dimethylaminopropyl)-3-ethylcarbodiimide (EDC) and 1-hydroxybenzotriazole (HOBt) in dimethylformamide (DMF) to give **B**. The Fmoc protecting group is removed with piperidine in DMF to yield free amine **C**. The next Fmoc-protected cyclic amino acid derivative **D** is coupled to **C** employing standard peptide (in this instance, 2-(1H-benzotriazol-1-yl)-1,1,3,3-tetramethyluronium hexafluorophosphate (HBTU), HOBt, and N,N-diisopropylethylamine (DIPEA) in DMF) to yield dipeptide **E**. The Fmoc group is removed with piperidine in DMF to yield the free amine **F**. A sulfonyl chloride derivative is reacted with **F** in the presence of DIPEA to yield **G**. The final product is removed from the resin with strong acid (in this instance, trifluoroacetic acid (TFA) in the presence of thioanisole and dithiane) to yield compounds of the present invention **H**.

Scheme 1.

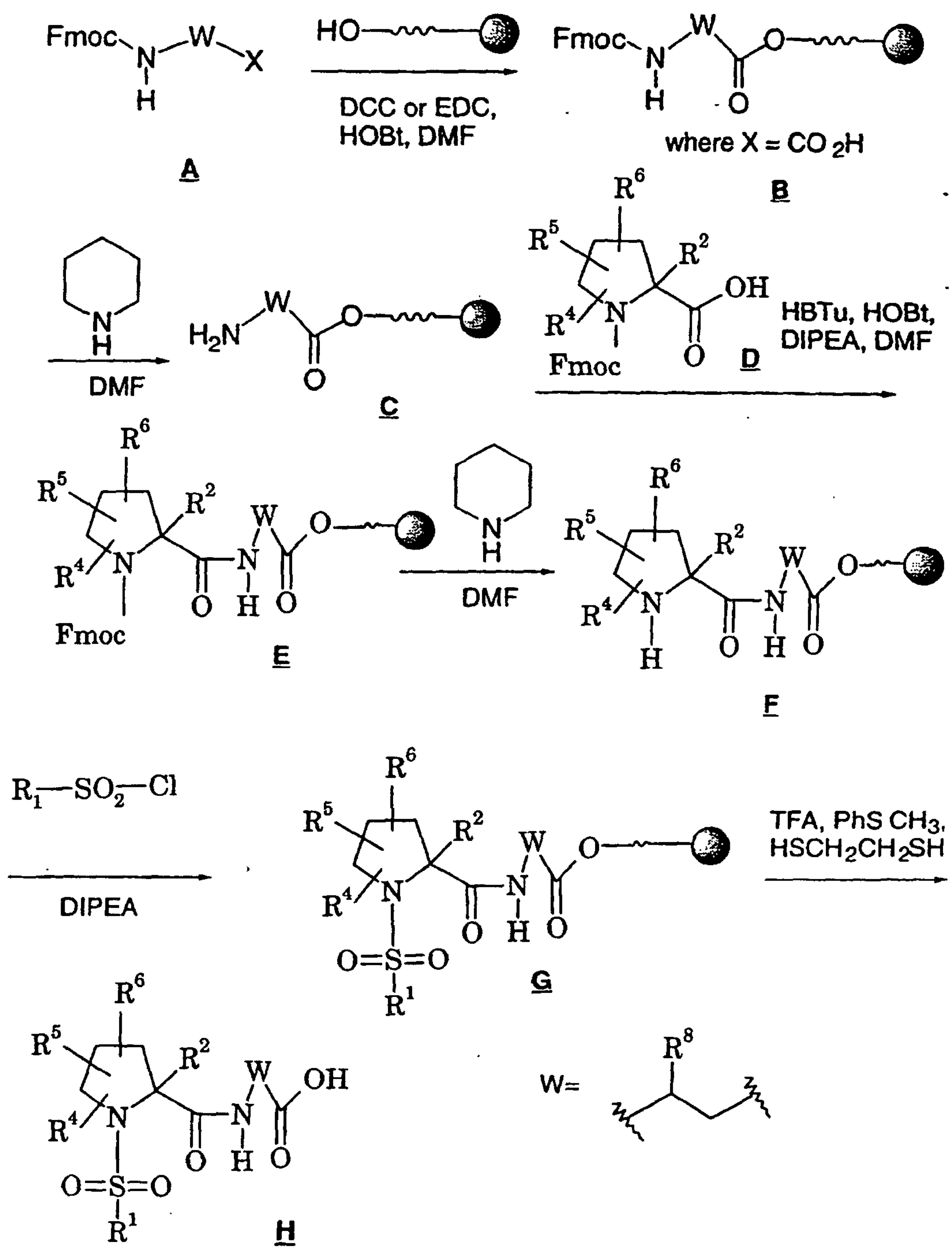


Compounds of the present invention may also be prepared by more traditional solution phase methodology outlined in Scheme 2. A N-*tert*-butyloxycarbonyl (t-Boc) protected cyclic amino acid derivative <u>**A**</u> is coupled to a acid-protected amino acid derivative <u>**B**</u> using a coupling agent such as dicyclohexylcarbodiimide (DCC) or 1-(3-dimethylaminopropyl)-3-ethylcarbodiimide (EDC) and 1-hydroxybenzotriazole (HOBt) and N-methylmorpholine (NMM) in methylene chloride ($CH_2Cl_2$) to give <u>**C**</u>. The t-Boc group is removed with hydrochloric acid in ethyl acetate to give the amine <u>**D**</u>. A sulfonyl chloride derivative is reacted with <u>**D**</u> in the presence of diethylamine and 4-dimethylaminopyridine (4-DMAP) to give <u>**E**</u>. The protecting group is removed from <u>**E**</u> employing an appropriate method to give <u>**F**</u>. Such methods would include a methyl ester being hydrolyzed with aqueous sodium hydroxide in ethanol (NaOH, EtOH), a benzyl

ester being removed by catalytic hydrogenation ($H_2$, $Pt_2O/C$, EtOH), an allyl ester being removed under catalytic conditions in the presence of aqueous diethylamine ($Pd(OAc)_2$, aq. DIEA) or a tert-butyl ester being removed with excess strong acid (trifluoroacetic acid (TFA) or hydrochloric acid (HCl)).

Scheme 2

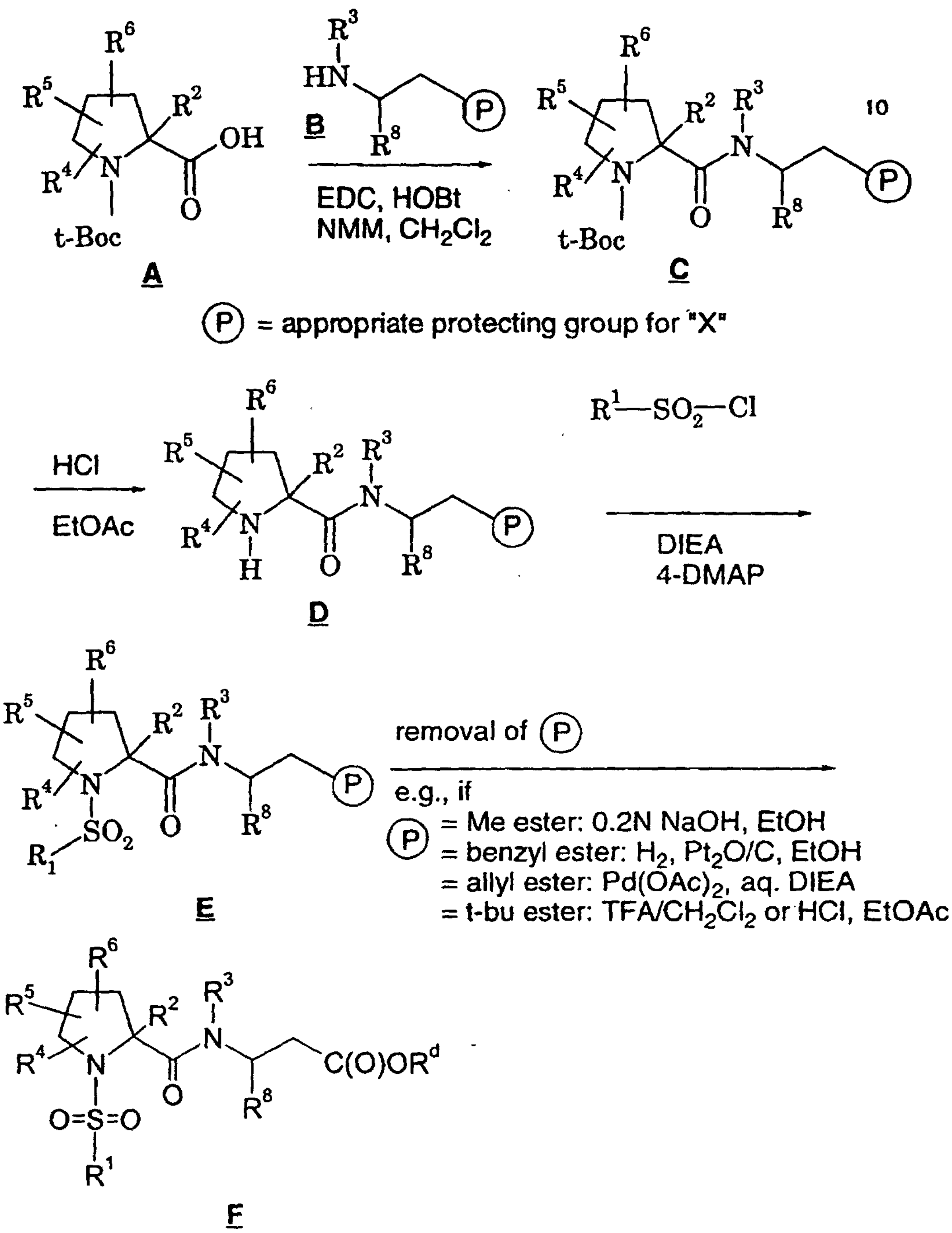


In Scheme 3, an appropriately protected β-aryl-β-alanine derivative <u>**A**</u> may be treated with triflic acid anhydride in the presence of pyridine to yield triflate <u>**B**</u>. Triflate <u>**B**</u> may be reacted with an aryl boronic acid under Suzuki reaction conditions or with a aryl-stannane derivative under Stille conditions to yield biaryl <u>**C**</u>. Triflate <u>**B**</u> may also be converted to an aryl-stannane derivative by reaction with hexamethylditin, tetrakis(triphenyl)palladium(0), triphenylphoshine, lithium chloride in hot dioxane to afford D. Aryl-stannane D may be reacted with an aryl halide under Stille conditions to afford C. C may be incorporated into the chemistry outlined in Schemes 1 and 2.

Scheme 3

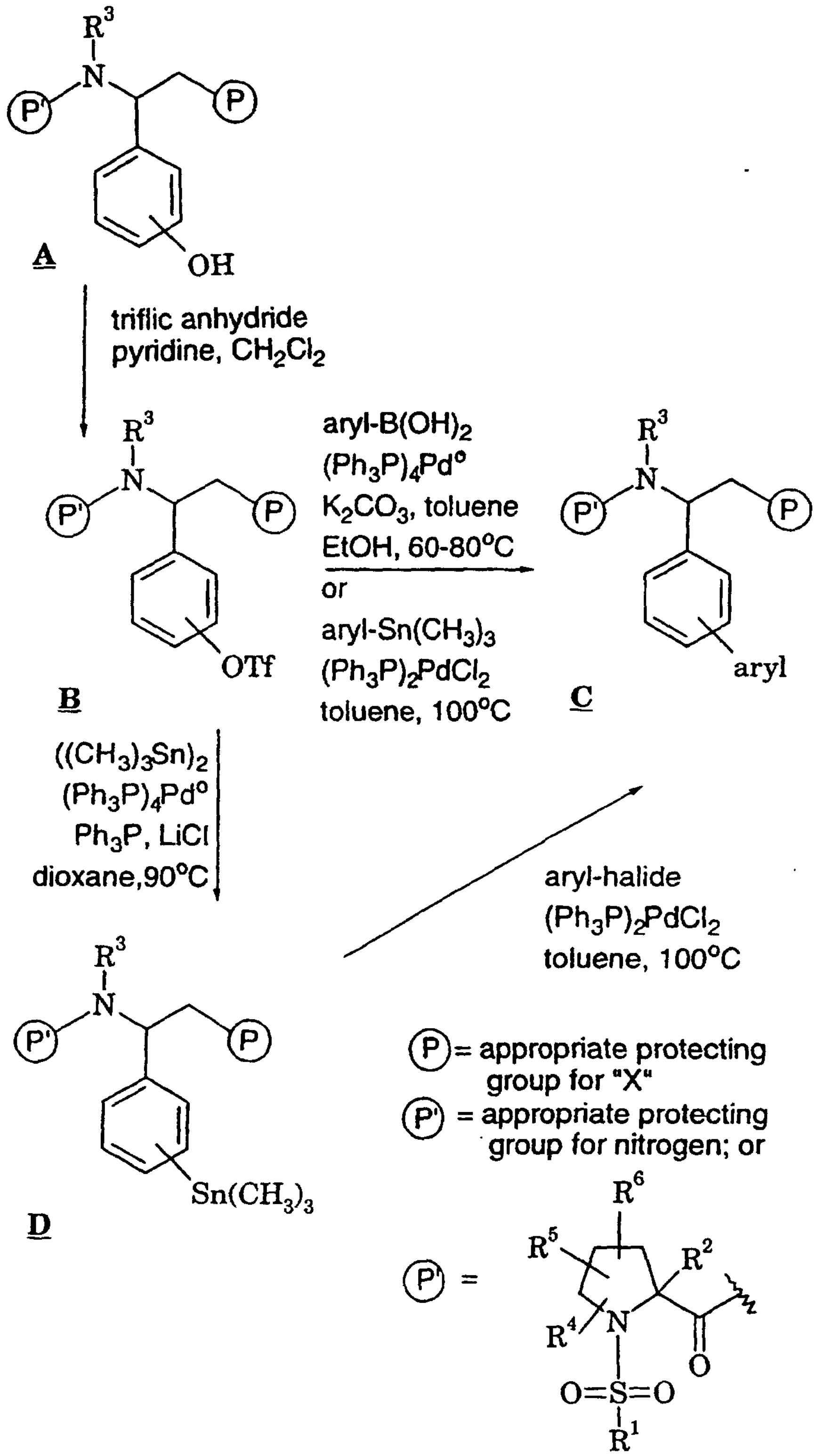

The following examples are provided to more fully illustrate the invention and are not to be construed as limiting the scope of the invention in any manner.

GENERAL PROCEDURE FOR THE SOLID-PHASE SYNTHESIS OF COMPOUNDS OF FORMULA I.

Step A. Loading of N-Fmoc-amino acid derivatives onto resins.

N-Fmoc-amino acids were loaded on either Wang® (Calbiochem-Novabiochem Corp.) or Chloro (2-chlorotrityl) resin. Wang® resin, typically 0.3 mmol, was washed with dimethylformamide three times. A solution of N-Fmoc-amino acid (0.3 mmol) in dimethylformamide (3 mL) was transferred to the pre-swollen Wang® resin. Dicyclohexylcarbodiimide (0.3 mmol) and 1-N-hydroxybenztriazole (0.3 mmol) was added and the mixture gently swirled for 2 hours. Following filtration, the resin was sequentially washed with dimethylformamide (3 times) and dichloromethane (3 times). The amino acid substitution value obtained after vacuum drying typically ranged between 0.07 to 0.1 mmol.

Alternatively, Chloro (2-chorotrityl) resin, typically 0.2 mmol, was pre-swollen in dimethylformamide. A solution of N-Fmoc-amino acid (0.2 mmol) in dimethylformamide (3 ml) was added to the resin, followed by the addition of N, N-diisopropylethylamine(0.4 mmol). The resin was gently stirred for 2 hours, filtered and washed sequentially with dimethylformamide (3 times) and dichloromethane (3 times). The resin was finally washed with 10% methanol in dichloromethane and vacuum dried. The amino acid substitution value obtained after vacuum drying typically ranged between 0.05 to 0.1 mmol.

Step B. Deprotection of the N-Fmoc group.

The N-Fmoc protecting group was removed from the resin from Step A by treatment with 20% piperidine in dimethylformamide for 30 minutes. Following filtration, the resin was washed sequentially with dimethylformamide (3 times), dichloromethane (1 time) and dimethylformamide (2 times) and used in the subsequent reaction.

Step C. Coupling of the next N-Fmoc-amino acid derivative

A solution of the next desired N-Fmoc-amino acid derivative (0.4 mmol) in dimethylformamide (2 mL) was mixed with 2-(1H-benzotriazol-1-yl)-1,1,3,3-tetramethyluronium hexafluorophosphate (0.4 mmol), 1-N-hydroxybenztriazole(0.4 mmol) and diisopropylethylamine (0.6 mmol). This solution was transferred to resin from Step B and typically allowed to react for 2 hours. Couplings were monitored by ninhydrin reaction. The coupling mixture was filtered and the resin washed with dimethylformamide (3 times) and used in the subsequent reaction.

Step D. Deprotection of the N-Fmoc group.

The N-Fmoc protecting group was removed from the resin from Step C by the procedure described in Step B and used in the subsequent reaction.

Step E. Sulfonylation of the terminal amino group.

The desired N-terminal capping reagent (sulfonylchloride) (0.4 mol) was dissolved in dimethylformamide (2 ml), mixed with N,N-diisopropylethylamine(0.8 mmol) and added to the resin from Step D. After approximately two hours, the resin was sequentially washed with dimethylformamide (3 times) and dichloromethane (3 times).

Step F. Cleavage of the desired products from the resins.

The final desired products were cleaved from the resins from Step E by gently stirring with a solution of trifluoroacetic acid:thioanisole:ethanedithiol (95:2.5:2.5); 3 hours for Wang® resin and 30 minutes for the Chloro (2-chorotrityl) resin. Following filtration, the solvents were removed by evaporation and the residue dissolved in acetonitrile (3 mL). Insoluble material was removed by filtration. The final products were purified by reverse phase chromatography with a linear gradient of buffer A (0.1% trifluoroacetic acid in water) and buffer B (0.1% trifluoroacetic acid in acetonitrile) and isolated by lyophilization. Molecular ions were obtained by electrospray ionization mass spectrometry or matrix-assisted laser desorption ionization time-of-flight mass spectrometry to confirm the structure of each peptide.

The following compounds were prepared by the general procedure described above using the appropriate amino acid and sulfonyl chloride derivatives:

EXAMPLE 1 and 2

N-(3,5-Dichlorobenzenesulfonyl)-2(S)-prolyl-3(R)-amino-3-(4-biphenyl)-propionic acid and N-(3,5-Dichlorobenzenesulfonyl)-2(S)-prolyl-3(S)-amino-3-(4-biphenyl)propionic acid

Step A. N-tert-Butoxycarbonyl-(S)-4-hydroxyphenylglycine

To a solution of (S)-(4-hydroxyphenyl)glycine (Sigma Chemical) ( (6.5 g, 39 mmol) in dioxane/water (1:1, 120 mL) was added triethylamine (5,9 g, 8.2 mL, 58 mmol) and [2-(tert-butoxycarbonyloxy-imino)-2-phenylacetonitrile] (BOC-ON; 11 g, 45 mmol). After stirring overnight at room temperature, 300 mL of brine was added to the solution and the mixture was extracted with ether. (3 x 100 mL). The aqueous layer was acidified with HCl (pH=2) and extracted with 3 x 100 mL of ethyl acetate. The ethyl acetate layer was dried over $MgSO_4$, filtered and the solvent removed under reduced pressure. The residue was chromatographed with 98/2 to 95/5 methylene chloride/methanol. Recovered 12 g of crude product. The impurity was removed following esterification of the product in the next step.
400 MHz $^1$H NMR ($CDCl_3$): δ 1.37 (s, 9H), 5.1 (1H, br s), 6.7 (d, 2H, J=8 Hz), 7.15 (d, 2H, J=8 Hz).

Step B. N-tert-Butoxycarbonyl-(S)-4-hydroxyphenylglycine, methyl ester

In a 50 mL round bottomed flask was added a 1:1 mixture of benzene:methanol and N-tert-butoxycarbonyl-(S)-4-hydroxyphenylglycine (2.8 g, 11 mmol). The solution was cooled to 0° C and a 2 M solution of trimethylsilyldiazomethane (Aldrich Chemical Co.) in hexane was added with vigorous stirring until a slight yellow color persisted. Then the reaction mixture solvents were removed under reduced pressure and the crude product was purified by flash chromatography (80/20 hexane/ethyl acetate) to give N-tert-butyloxycarbonyl-(S)-4-hydroxyphenylglycine, methyl ester (2.05 g, 7.3 mmol) (66% yield).
300 MHz $^1$H NMR ($CDCl_3$): δ 1.43 (s, 9H), 3.71 (s, 3H), 5.22 (br d, 1H), 5.57 (1H, br d), 5.80 (br s, 1H), (6.7 (d, 2H, J=8 Hz), 7.17 (d, 2H, J=8 Hz).

Step C. N-*tert*-Butoxycarbonyl-(S)-4-trifluoromethylsulfonyloxyphenylglycine, methyl ester

To a 25 mL round bottom flask fitted with a stir bar and septum was added N-tert-butyloxycarbonyl-(S)-4-hydroxyphenylglycine, methyl ester (1.9 g, 6.8 mmol) and pyridine (2.8 mL, 33 mmol) in 12 mL of methylene chloride. The flask was purged with $N_2$, cooled to 0° and trifluoromethanesulfonic anhydride (1.38 mL, 7.8 mmol) was added dropwise over several minutes, keeping the temperature at or below 4° C. The solution was stirred for 1 h, then at room temperature for 4 h. The mixture was diluted with 20 mL of methylene chloride. The mixture was washed with 20 mL of 0.5 N NaOH, 1 x 20 mL of water and 2 x 20 mL of 10% citric acid. Dry the organic layer over $MgSO_4$, filter, reduce the volume. Flash chromatography (75/25 hexane/methylene chloride) gave 2.3g of desired product ( 81% yield).
300 MHz $^1$H NMR ($CDCl_3$): δ 1.43 (s, 9H), 3.74 (s, 3H), 5.35 (1H, br d), 5.68 (br s, 1H), 7.27 (d, 2H, J=8 Hz), 7.47 (d, 2H, J=8 Hz).

Step D. N-tert-Butoxycarbonyl-(S)-(4-biphenyl)glycine.

To a 25 mL round bottom flask fitted with a stir bar and septum was added N-tert-butyloxycarbonyl-(S)-4-trifluoromethylsulfonyloxyphenylglycine, methyl ester (690 mg, 1.67 mmol), anhydrous potassium carbonate (348 mg, 2.6 mmol) and benzeneboronic acid (411 mg, 3.4 mmol) in 15 ml of toluene and 3 mL of ethanol. The mixture was degassed under nitrogen with three freeze-thaw cycles and tetrakis(triphenylphosphine) palladium (94 mg, 0.085 mmol) was added to the reaction mixture and the mixture was heated between 75-90° C for 4 h. The solvent was removed under reduced pressure and the residue flash chromatographed with 85/15 hexane/ethyl acetate. Recovered 600 mg of the methyl ester (quantitative yield).
300 MHz $^1$H NMR ($CDCl_3$): δ 1.44 (s, 9H), 3.75 (s, 3H), 5.37 (1H, br d), 5.62 (br s, 1H), 7.36 (m,.1H), 7.45 (m, 4H), 7.57 (m, 4H).
The ester was hydrolyzed with 1.2 eq of KOH in 10 mL of 4:1 ethanol: water (2 h). The solution was acidified with 2 N HCl (pH=2). Remove the solvents in vacuo and extract the free acid with methylene chloride. Recovered 430 mg of free acid (66% yield).

Step E. 3-(N-*tert*-Butyloxycarbonyl)amino-1-diazo-3-(4-biphenyl)propan-2-one.

To a 25 mL round bottom flask fitted with a stir bar and septum was added N-tert-butoxycarbonyl-(S)-4-biphenylglycine (430 mg, 1.31 mmol) in 10 mL of 2:1 methylene chloride: ether. The mixture was cooled to 0° C and N-methylmorpholine (159 µl, 1.44 mmol) was added, followed by dropwise addition of isobutylchloroformate (179 µl, 1.38 mmol). The mixture was stirred for 1 h at 0° C, then diazomethane in ether (excess, prepared from Diazald$^R$ by literature procedure) was added dropwise to the reaction mixture. The mixture was stirred for 1 h then quenched with saturated sodium bicarbonate. The mixture was extracted with ethyl acetate. (2 x 5 mL), washed with brine then dried over $MgSO_4$. The mixture was filtered, the solvent removed under reduced pressure and the product isolated by flash chromatography (80/20 hexane/ethyl acetate) to give 280 mg (0.78 mmol) of product (58% yield).
300 MHz $^1$H NMR ($CDCl_3$): δ 1.42 (s, 9H), 5.22 (bs, 1H), 5.29 (s, 1H), 5.9 (br s, 1H), 7.35-7.5 (m, 5H), 7.52-7.62 (m, 4H).

Step F. 3(R)-amino-3-(4-biphenyl)propionic acid, methyl ester

To a 25 mL round bottom flask fitted with a stir bar and septum was added (3-diazo-2-oxopropyl-1-(S)-(4-biphenyl))carbamic acid,tert-butyl ester (280 mg, 0.76 mmol),with 5 mL each of methanol and dioxane. The flask was cooled to 0 °C and 0.15 eq (34 mg, 0.038 mmol) of silver benzoate in 500 µl of triethylamine was added dropwise to the reaction mixture and the mixture allowed to stir at 25° C for 1 h. The reaction was worked up with 10% $NH_4OH$ in saturated $NH_4Cl$ (10 mL). Extract with ether (3 x 10 mL) and dry the organic layer over $MgSO_4$. Filter, reduce the volume and flash chromatograph with 85/15 hexane/ethyl acetate. Recovered 260 mg of product (98% yield). Take this material and dissolve it in 10 mL of 1 N HCl in ethyl acetate. After stirring 2 h at room temperature, we obtained 180 mg of 3(R)-amino-(4-biphenyl)propionic acid, methyl ester hydrochloride. 300 MHz $^1$H NMR ($CD_3OD$): δ 2.90 (dd, 1H, J=18 Hz, J=6 Hz), 3.02 (dd, 1H, J=18 Hz, J=6 Hz), 3.66 (s, 3H), 5.9 (br s, 1H), 7.33-7.5 (m, 5H), 7.55-7.6 (m, 4H).

Step G. N-(3,5-Dichlorobenzenesulfonyl)-(L)-proline

To a mixture of (L)-proline methyl ester hydrochloride (838 mg, 5.06 mmol) in methylene chloride (25 mL) at 0°C were added N,N-diisopropylethylamine (2.64 mL, 15.2 mmol) and a solution of 3,5-dichlorobenzenesulfonyl chloride (1.49 g, 6.07 mmol) in methylene chloride (5 mL). The cooling bath was removed, and the mixture was stirred overnight at room temperature. It was then diluted with methylene chloride, washed with 1N hydrochloric acid, saturated $NaHCO_3$, saturated brine solution, dried ($Na_2SO_4$), and evaporated. The methyl ester was obtained pure by silica gel chromatography eluting with 10% acetone in hexane; yield 1.49 g. It was then taken up in ethanol (50 mL) and treated with 0.2 N sodium hydroxide (26.6 mL) for 1.5 hours at room temperature. The mixture was acidified with glacial acetic acid, concentrated, the residue taken up in methylene chloride, washed with water, saturated brine solution, dried ($Na_2SO_4$), and evaporated to give the title compound; yield 1.4 g.
400 MHz $^1$H NMR ($CD_3OD$): δ 1.80-2.15 (m, 4H); 3.35-4.45 (m, 2H); 4.30 (dd, 1H); 7.76 (m, 1H); 7.83 (m, 2H).

Step H. N-(3,5-Dichlorobenzenesulfonyl)-2(S)-prolyl-3(R)-amino-3-(4-biphenyl)propionic acid, methyl ester and N-(3,5-dichlorobenzenesulfonyl)-2(S)-prolyl-3(S)-amino-3-(4-biphenyl)propionic acid, methyl ester.

To a 10 mL round bottom flask fitted with a stir bar and septum was added 3(R)-amino-3-(4-biphenyl)propionic acid (92 mg, 0.36 mmol), N-methylmorpholine (99 µl, 0.7 mmol), 1-hydroxybenzotriazole hydrate.(75 mg, 0.55 mmol) and N-(3,5-dichlorobenzenesulfonyl)-2(S)-proline (125 mg, 0.43 mmol) in 5 ml of methylene chloride. Then 1-ethyl-3-(3-dimethylaminopropyl)carbodiimide hydrochloride (83 mg, 0.43 mmol) was added and the mixture stirred overnight at 24° C. The reaction mixture was worked up by adding 0.5 N HCl (pH=3) and extracting with methylene chloride. The solvent was removed and the residue flash chromatographed (70/30) to give two products: 60 mg of the higher Rf product N-(3,5-dichlorobenzenesulfonyl)-2(S)-prolyl-3(R)-amino-3-(4-biphenyl)propionic acid, methyl ester.
400 MHz $^1$H NMR ($CDCl_3$): δ 1.7-1.9 (m, 4H), 2.2-2.3 (bs, 1H), 2.9-3.1 (m, 2H), 3.1-3.3 (m, 1H), 3.65 (s, 3H), 4.05-4.15 (m, 2H), 5.4-5.5 (m, 1H), 7.22 (m, 1H), 7.3-7.5 (m, 4H), 7.55 (m, 4H), 7.72 (d, 1H, J=6Hz), 7.8 (m, 1H); and 60 mg of the lower Rf product N-(3,5-dichlorobenzenesulfonyl)-2(S)-prolyl-3(S)-amino-3-(4-biphenyl)propionic acid, methyl ester.
400 MHz $^1$H NMR ($CDCl_3$): δ 1.7-1.9 (m, 5H), 2.2-2.3 (bs, 1H), 2.9 (d, 2H, J=8 Hz), 3.1-3.3 (m, 1H), 3.65 (s, 3H), 4.08-4.16 (m, 1H), 5.4-5.5 (m, 1H), 7.25-7.35 (m, 1H), 7.4(bd, 4H), 7.55 (bd, 3H), 7.71 (m, 3H).

Step I. N-(3,5-Dichlorobenzenesulfonyl)-2(S)-prolyl-3(R)-amino-3-(4-biphenyl)propionic acid, and N-(3,5-dichlorobenzenesulfonyl)-2(S)-prolyl-3(S)-amino-3-(4-biphenyl)propionic acid

Each of the components described in Step H was hydrolyzed separately to the free acid by adding to each 2 equivalents of KOH in 3/1 ethanol/ water. The solutions were acidified with 2.5 N HCl and each component was extracted with methylene chloride. Forty five mg of the higher Rf product N-(3,5-dichlorobenzenesulfonyl)-2(S)-prolyl-3(R)-amino-3-(4-biphenyl)propionic acid was recovered.

400 MHz $^1$H NMR ($CDCl_3$): δ 1.75 (m, 1H), 2.0 (m, 3H), 2.9-3.1 (m, 2H), 3.2 (m, 1H), 3.60 (m, 1H), 4.2 (m,1H), 5.4-5.5 (m, 1H), 7.3 (m, 1H), 7.41(m, 2H), 7.46 (d, 1H, J=2 Hz), 7.48 (d, 1H, J=2 Hz), 7.60 (t, 1H, J=2 Hz), 7.60 (t, 1H, J=2 Hz), 7.79 (d, 1H, J=2 Hz), 7.87, (t, 2H, J=2 Hz). 8.7 (d, 1H, J=9 Hz).
MS: m/e 565(M + H + $NH_3$).

Thirty mg of the lower Rf product N-(3,5-dichlorobenzenesulfonyl)-2(S)-prolyl-3(S)-amino-3-(4-biphenyl)propionic acid was recovered.
400 MHz $^1$H NMR ($CDCl_3$): δ 1.75 (m, 1H), 2.0 (m, 3H), 2.87 (d, 2H, J= 6Hz), 3.2 (m, 1H), 3.60 (m, 1H), 4.2 (m,1H), 5.35-5.45 (m, 1H), 7.3 (t, 1H, J=6 Hz), 7.41(t, 2H, J=6 Hz), 7.46 (d, 2H, J=6 Hz), 7.59 (d, 4H, J=8 Hz), 7.79 (d, 1H, J=2 Hz), 7.87, (d, 2H, J=2 Hz). 8.67 (d, 1H, J=9 Hz).
MS: m/e 565(M + H + $NH_3$).

EXAMPLE 3 and 4

N-(3,5-Dichlorobenzenesulfonyl)-2(S)-prolyl-3(R)-amino-3-(4-(2'-methoxyphenyl)phenyl)propionic acid and N-(3,5-dichlorobenzenesulfonyl)-2(S)-prolyl-3(S)-amino-3-(4-(2'-methoxyphenyl)phenyl)propionic acid

Step A. N-tert-Butoxycarbonyl-(S)-4-(2'-methoxyphenyl)phenylglycine.

The title compound was synthesized by the procedure described in Example 1 and 2, Step D by coupling N-(tert-butoxycarbonyl)-(S)-4-(trifluoromethylsulfonyloxy)phenylglycine, methyl ester (413 mg. 1.0 mmol) with 2-methoxybenzeneboronic acid (304 mg, 2.0 mmol) to provide 310 mg of the methyl ester product (81% yield).
300 MHz $^1$H NMR ($CDCl_3$): δ 1.45 (s, 9H), 3.74 (s, 3H), 3.81 (s, 3H), 5.42 (bd, 1H), 5.55 (bs, 1H), 5.7 (br s, 1H), 6.95-7.05 (m, 1H), 7.25-7.3 (m 3H), 7.4 (d, 1H, J=8 Hz), 7.48-7.52 (m, 3H).

This material was hydrolyzed to the free acid and used without further purification in the next step.

Step B. 3(S)-(N-*tert*-Butyloxycarbonyl)amino-1-diazo-3-(4-(2'-methoxyphenyl)phenyl)-propan-2-one.

The title compound was synthesized by the procedure described in Example 1 and 2, Step E by transforming N-tert-butoxycarbonyl-(S)-4-(2'-methoxyphenyl)phenylglycine (220 mg, 0.62 mmol) to the methyldiazoketone via the Arnt-Eistert reaction to provide120 mg (51% yield) of the homologated methyl ester.
400 MHz $^1$H NMR ($CDCl_3$): δ 1.41 (bs, 9H), 3.79 (s, 3H), 5.22 (bs, 1H), 5.29 (s, 1H), 5.85 (br s, 1H), 6.95-7.05 (m, 2H), 7.25-7.35 (m 4H), 7.5 (d, 2H, J=9 Hz).

Step C. 3(R)-amino-3-(4-(2'methoxyphenyl)phenyl)propionic acid, methyl ester hydrochloride

The title compound was synthesized by the procedure described in Example 1 and 2, Step F by effecting a Wolff rearrangement on (3-diazo-2-oxopropyl-1-(S)-(4-(2'methoxyphenyl)phenyl))carbamic acid, tert butyl ester (120 mg, 0.31 mmol) to give homologated Boc-β-aminoacid methyl ester.
400 MHz $^1$H NMR ($CDCl_3$): δ 1.41 (bs, 9H), 2.8-2.9 (m, 2H), 3.62 (s, 3H), 3.79 (s, 3H), 5.10 (bs, 1H), 5.45 (bs, 1H), 5.85 (br s, 1H), 6.95-7.05 (m, 2H), 7.25-7.35 (m 4H), 7.48 (d, 2H, J=9 Hz).

This material was dissolved in 10 mL of 1 N HCl in ethyl acetate. After stirring 2 h at room temperature, 45 mg of 3(R)-amino-3-(4-(2'-methoxy)-biphenyl)propionic acid, methyl ester hydrochloride was obtained. This material was carried on to the next step without further characterization.

Step D: N-(3,5-Dichlorobenzenesulfonyl)-2(S)-prolyl-3(R)-amino-3-(4-(2'-methoxyphenyl)phenyl)propionic acid, methyl ester and N-(3,5-dichlorobenzenesulfonyl)-2(S)-prolyl-3(S)-amino-3-(4-(2'-methoxyphenyl)phenyl)propionic acid, methyl ester

The title compounds were synthesized by the procedure described in Example 1 and 2, Step H by effecting the coupling reaction of 3(R)-amino-3-(4-(2'-methoxyphenyl)phenyl)propionic acid hydrochloride (48 mg, 0.13 mmol) with N-(3,5-dichlorobenzenesulfonyl)-2(S)-proline (49 mg, 0.15 mmol). Two products were obtained: 33 mg of the higher Rf product N-(3,5-dichlorobenzenesulfonyl)-2(S)-prolyl-3(R)-amino-3-(4-(2'-methoxybiphenyl)propionic acid, methyl ester.
400 MHz $^1$H NMR ($CDCl_3$): δ 1.7-1.9 (m, 3H), 2.2-2.3 (bs, 1H), 2.92 (dd, 1H, J=16 Hz, J=6 Hz), 3.02 (dd, 1H, J=16 Hz, J=6 Hz), 3.1-3.2 (m, 1H), 3.65 (m, 1H), 3.67 (s, 3H), 3.80 (s, 3H), 4.05-4.15 (m, 2H), 5.4-5.5 (m, 1H), 6.9-7.0 (m, 2H), 7.3-7.35 (m, 3H), 7.50 (d, 2H, J=8 Hz), 7.60 (t, 1H, J= 2 Hz), 7.72 (d, 1H, J=1 Hz), 7.8 (m, 1H);
and 11 mg of the lower Rf product N-(3,5-dichlorobenzenesulfonyl)-2(S)-prolyl-3(S)-amino-3-(4-(2'-methoxyphenyl) phenyl)propionic acid, methyl ester.

400 MHz $^1H$ NMR ($CDCl_3$): δ 1.7-1.9 (m, 3H), 2.2-2.3 (bs, 1H), 2.83 (d, 1H, J= 6 Hz), 2.9 (d, 1H, J=8 Hz), 3.1-3.2 (m, 1H), 3.67 (s over m, 4H), 3.79 (s, 3H), 4.08-4.16 (m, 1H), 5.4-5.5 (m, 1H), 6.9-7.0 (m, 2H), 7.15 (d, 1H, J=9 Hz), 7.25-7.3 (m, 2H), 7.35 (m, 2H), 7.50 (d, 2H, J=8 Hz), 7.60 (t, 1H, J= 2 Hz), 7.72 (d, 1H, J=1 Hz).

Step E. N-(3,5-Dichlorobenzenesulfonyl)-2(S)-prolyl-3(R)-amino-3-(4-(2'-methoxyphenyl)phenyl)propionic acid and N-(3,5-dichlorobenzenesulfonyl)-2(S)-prolyl-3(S)-amino-3-(4-(2'-methoxyphenyl)phenyl)propionic acid.

Each of the components described in Step D. was hydrolysed separately to the free acid by adding to each 2 equivalents of KOH in 3/1 ethanol/ water. The solutions were acidified with 2.5 N HCl and each component was extracted with methylene chloride. Each component was flash chromatographed using 97/3/0.2 methylene chloride/ methanol/ acetic acid. Twenty mg of the higher Rf product N-(3,5-dichlorobenzenesulfonyl)-2(S)-prolyl-3(R)-amino-3-(4-(2'-methoxyphenyl)phenyl)propionic acid was recovered.
400 MHz $^1H$ NMR ($CD_3OD$): δ 1.7-1.9 (m, 4H), 2.8-2.95 (m, 2H), 3.65 (m, 1H), 3.77 (s, 3H), 4.05-4.15 (m, 2H), 5.4-5.5 (m, 1H), 6.95-7.05 (m, 2H), 7.25-7.30 (m, 2H), 7.4-7.5 (m, 4H), 7.78 (t, 1H, J=1 Hz), 7.86 (d, 1H, J=2 Hz).
MS: m/e 594(M+1+$NH_3$).

Six mg of the lower Rf product N-(3,5-dichlorobenzenesulfonyl)-2(S)-prolyl-3(S)-amino-3-(4-(2'-methoxyphenyl)phenyl)propionic acid was recovered.
400 MHz $^1H$ NMR ($CD_3OD$): δ 1.7-1.8 (m, 1H), 2.0 (m, 2H), 2.86 (d, 1H, J= 13 Hz), 3.3-3.4 (m, 1H), 3.5-3.6 (m, 1H), 3.77 (s, 3H), 4.25 (m, 1H), 5.4-5.5 (m, 1H), 6.9-7.0 (m, 2H), 7.25-7.3 (m, 2H), 7.40 (d, 2H, J=8 Hz), 7.48(d, 2H, J=8 Hz), 7.72 (d, 1H, J=1 Hz), 7.80 (d, 2H, J=1 Hz).
MS: m/e 594(M +1 + $NH_3$).

PREPARATIVE EXAMPLE 1

N-(3,5-Dichlorobenzenesulfonyl)-2(S)-prolyl-3(R)-amino-3-(4-*tert*-butyloxyphenyl)propionic acid.

Step A. 4-Benzyloxyphenyldiazoniumtetrafluoroborate.

In a 250 mL round bottomed flask fitted with a stir bar was added 4-benzyloxyaniline (8.7g, 43.6 mmol), 150 mL of ethanol and 17 mL of 48% fluoroboric acid. Cool to 0° C. Then isoamyl nitrite (6,64 mL, 50 mol) was added dopwise over 15 minutes, keeping the solution temperature below 8° C. Stir 2 h at 0-4° C. The product precipitated out of solution. Diluted the reaction mixture with 100 mL ether and filter the reaction mixture. Wash the precipitate with 2 x 50 mL of ether. Recovered 10.3 g (79%) of product. Melting point =137° (dec), Lit.=140-142 (dec).

Step B. 4-Benzyloxycinnamic acid, methyl ester

The following reaction was adapted from M. Beller and K. Kuhlein, *Synlett*, p 441 (1995). In a 50 mL round bottomed flask fitted with a stir bar and septum was added 4-benzyloxyphenyl-diazoniumtetrafluoroborate (3.o g, 10.2 mmol) and methyl acrylate (1.72 g, 0 mmol) in 15 mL of methanol. Subsequently, 10% palladium on carbon (250 mg, 0.2 mmol) was added to the mixture and it was heated at 55-60° C until nitrogen gas evolution ceased (2 h) then overnight at 50° C. The reaction was cooled to room temperature, the catalyst filtered off and washed with methanol. The solvent is removed under reduced pressure and the residue purified by flash chromatography (90/10 hexane/ethyl acetate) Recovered 2.0 g of product (70% yield).
400 MHz $^1H$ NMR ($CDCl_3$): δ 3.78 (s, 3H), 5.08 (s, 2H), 6.25 (d, 1H, J= 17 Hz), 6.29 (d, 1H, J=9 Hz), 7.3-7.4 (m, 5H,), 7.45 (d, 2H, J= 9 Hz), 7.62 (d, 1H, J= 14 Hz).

Step C. 3-(4-benzyloxyphenyl)-3(R)-[benzyl-(1(S)-phenylethyl)-amino]propionic acid, methyl ester

This procedure is was adapted from S.G. Davies and O. Ichihara, *Tetrahedron: Asymmetry,* **2**, p 183 (1991). In a 100 mL round bottom flask fitted with a stir bar and rubber septum is added (S)-(-)-N-benzyl-1-phenylethylamine (1.69 g, 8.0 mmol) in 60 mL of anhydrous tetrahydrofuran. Cooled to 0° C and flushed with nitrogen. n-Butyl lithium (2.5N solution in hexane, 3.2 mL) was added dropwise, keeping the temperature below 4° C for 15 minutes after final base addition. Then cooled to -78 ° C and slowly added 4-benzyloxycinnamic acid, methyl ester (1.07g, 4.0 mmol) in 15 ml of dry tetrahydrofuran at such a rate that the solution temperature remaines below -60° C. Stirred for 15 minutes, then quenched with saturated ammonium chloride (5 mL). Warmed to room temperature and added 10 mL of saturated brine. Extracted with 2 x 25 mL of ether, dried over $MgSO_4$. Filtration and evaporation gave a mixture of the adduct and excess amineas a pale yellow oil. Flash chromatography (90/10 hexane/ethyl acetate) gave the product (1.25 g, 2.62 mmol) (66% yield) which ran just above the excess amine on TLC).

400 MHz $^1$H NMR ($CDCl_3$): δ1.19 (d, 2H, J=7 Hz), 2.50 ( dd, 1H, J=13 Hz, J=10 Hz), 2.64 (dd, 1H J=13 Hz, J=6 Hz), 3.44 (s, 3H), 3.62 (q, 2H, J= 15 Hz), 3.97 (q, 1H, J= 6 Hz), 4.36 (dd, 1H, J= 9 Hz, J=6 Hz), 5.03 (S, 2H), 6.93 (d, 2H, J=9 Hz), 7.2-7.5 (m, 17 H).

Step D. 3(R)-amino-3-(4-hydroxphenyl)propionic acid, methyl ester, acetic acid salt

To a 250 mL medium pressure Parr hydrogenation bottle was added 25 mL of methanol, 1 mL of glacial acetic acid, 100 mg of 10% palladium hydroxide on carbon and 3-(4-benzyloxyphenyl)-3(R)-[benzyl-(1(S)-phenylethyl)-amino] propionic acid, methyl ester(1.25 g, 2.6 mmol). The flask was evacuated then pressurized to 50 psi $H_2$. and shaken until no more $H_2$ uptake was observed (4 h). Filter the solution through Celite, wash the pad with methanol (50 mL) and concentrate the filtrate under reduced pressure. Recovered 660 mg of product (theoretical) which was used without further purification.

Step E. N-(3,5-Dichlorobenzenesulfonyl)-(L)-proline, pentafluorophenol ester

To a 50 mL round bottomed flask fitted with a stir bar and septum was added N-(3,5-dichlorobenzenesulfonyl) -(L)-proline (from Example 1, Step G) (680 mg, 2.10 mmol) and 10 mL of ethyl acetate. Then dicyclohexylcarbodiimide (563 mg, 2.7 mmol) and pentafluorophenol (1.1 g, 6.0 mmol) were added to the flask and the mixture stirred for 2 h. The urea was filtered off and washed with 2 x 15 mL of ethyl acetate. The residue was used subsequently without purification. TLC (70/30 hexane/ethyl acetate) indicated that no starting material remained.

Step F. N-(3,5-Dichlorobenzenesulfonyl)-2(S)-prolyl-3(R)-amino-3-(4-hydroxyphenyl)propionic acid,methyl ester

To a 50 mL round bottomed flask was added crude N-(3,5-dichlorobenzenesulfonyl)-(L)-proline, pentafluorophenol ester in 2/1 dioxane/methylene chloride (30 mL) and 3(R)-amino-3-(4-hydroxyphenyl)propionic acid, methyl ester (500 mg, 2.56 mmol, from Example 5, Step C). The suspension was heated with stirring over 20 min to 55 ° C, then overnight at 40° C. The reaction mixture was worked up by dissolving the residue in 50 mL of methylene chloride and extracting it with 4 x 25 mL of saturated sodium bicarbonate,dried over $MgSO_4$, filtered and the sovent removed under reduced pressure. The residue was flash chromatographed (85/15 hexane/ ethyl acetate) and N-(3,5-dichlorobenzenesulfonyl)-2(S)-prolyl-3(R)-amino-3-(4- hydroxyphenyl)-propionic acid,methyl ester (800 mg, 1.5 mmol) was recovered (76% yield). 400 MHz $^1$H NMR ($CDCl_3$): δ 1.6-1.75 (m, 3H), 2.15-2.25 (bs, 1H), 2.85 (dd, 1H, J=16 Hz, J=6 Hz), 2.95 (dd, 1H, J=16 Hz, J=6 Hz), 3.15-3.25 (m, 1H), 3.63 (m, 1H), 3.66 (s, 3H), 4.10-4.15 (m, 1H), 5.35-5.45 (m, 1H), 6.73 (d, 2H, J=8 Hz), 7.12 (d, 2H, J=8 Hz), 7.61 (t, 1H, J= 2 Hz), 7.71 (d, 1H, J=1 Hz), 7.73 (m, 1H).

Step G. N-(3,5-Dichlorobenzenesulfonyl)-2(S)-prolyl-3(R)-amino-3-(4-tert-butyloxyphenyl)propionic acid, methyl ester

In a 500 μl spin vane vial fitted with a magnetic stirrer was added N-(3,5-dichlorobenzenesulfonyl)-2(S)-prolyl-3(R)-amino-3-(4-hydroxyphenyl)propionic acid,methyl ester (25 mg, 0.05 mmol), tert-butyloxytrichoroacetimidate (12 mg, 0.055 mmol) and 300 μl of a 2/1 mixture of cyclohexane/methylene chloride. Then a catalytic amount of boron trifluoride etherate (5 μl) was added and the reaction was stirred at 24° C for 1 h. No starting material was seen by TLC (70/30 hexane/ethyl acetate). The reaction was worked up with 1 mL of saturated sodium bicarb and 2 mL of methylene choride, dried over $MgSO_4$, filtered and the solvent removed under reduced pressure. Flash chromatography (70/30 hexane/ethyl acetate) afforded 25 mg of product (89% yield).
400 MHz $^1$H NMR ($CDCl_3$): δ 1.33 (s, 9H,), 1.7-1.8 (m, 3H), 2.15-2.25 (bs, 1H), 2.87 (dd, 1H, J=16 Hz, J=6 Hz), 2.97 (dd, 1H, J=16 Hz, J=6 Hz), 3.15-3.25 (m, 1H), 3.63 (m, 1H), 3.62 (s, 3H), 4.10-4.15 (m, 1H), 5.35-5.45 (m, 1H), 6.95 (d, 2H, J=8 Hz), 7.18 (d, 2H, J=8 Hz), 7.61 (t, 1H, J= 2 Hz), 7.71 (d, 1H, J=1 Hz), 7.73 (m, 1H).

Step H. N-(3,5-Dichlorobenzenesulfonyl)-2(S)-prolyl-3(R)amino-3-(4-tert-butyloxyphenyl)propionic acid

The product described in Step G. was hydrolysed to the free acid by adding 2 equivalents of KOH in 3/1 ethanol/ water. The solution was acidified with 2.5 N HCl and extracted with methylene chloride. The product was flash chromatographed using 97/3/0.2 methylene chloride/methanol/acetic acid. Recovered 16mg of product (66 % yield). 400 MHz $^1$H NMR ($CD_3OD$): δ 1.31 (s, 9H,), 1.7-1.8 (m, 1H), 1.9-2.0( m, 3H), 2.81 (m, 2H), 3.2-3.3 (m, 2H), 3.5-3.6 (m, 1H), 4.2 (m, 1H), 5.35-5.45 (m, 1H), 6.95 (d, 2H, J=8 Hz), 7.30 (d, 2H, J=8 Hz), 7.7-7.8 (m, 3H), 8.75 (m, 1H). MS: m/e 560 (M +1 + $NH_3$).

EXAMPLE 5

N-(3,5-Dichlorobenzenesulfonyl)-2(S)-prolyl-3(R)-amino-3-(4-(2'-cyanophenyl)phenyl)propionic acid

Step A. N-(3,5-Dichlorobenzenesulfonyl)-2(S)-prolyl-3(R)-amino-3-(4-trifluoromethylsulfonyloxyphenyl)propionic acid) propionic acid, methyl ester

The title compound was made according to the procedure described in Example 1 and 2, Step C starting with N-(3,5-dichlorobenzenesulfonyl)-2(S)-prolyl-3(R)-amino-3-(4-hydroxyphenyl)propionic acid,methyl ester (500 mg, 1.0 mmol) (from Preparative Example 1, Step F) to provide 400 mg (67% yield) of desired product.
400 MHz $^1H$ NMR ($CDCl_3$): δ 1.6-1.8 (m, 3H), 2.15-2.25 (bs, 1H), 2.2 (dd, 1H, J=16 Hz, J=6 Hz), 2.94 (dd, 1H, J=16 Hz, J=6 Hz), 3.15-3.25 (m, 1H), 3.63 (m, 1H), 3.67 (s, 3H), 4.10-4.15 (m, 1H), 5.4-5.5 (m, 1H), 6.95 (d, 2H, J=8 Hz), 7.26 (d, 2H, J=3 Hz), 7.40 (d, 2H, J=9 Hz), 7.61 (t, 1H, J= 2 Hz), 7.71 (d, 2H, J=1 Hz), 7.91 (m, 1H).

Step B. N-(3,5-Dichlorobenzenesulfonyl)-2(S)-prolyl-3(R)-amino-3-(4-(2'-cyanophenyl)phenyl)propionic acid, methyl ester

The title compound was made according to the procedure described in Example 1 and 2, Step D starting with N-(3,5-dichlorobenzenesulfonyl)-2(S)-prolyl-3(R)-amino-3-(4-trifluoromethylsulfonyloxyphenyl)propionic acid, methyl ester (40 mg, 0.067mmol) and 2-cyanobenzene boronic acid (15 mg, 0.10 mmol) to provide 15 mg (38% yield) of desired product.
400 MHz $^1H$ NMR ($CDCl_3$): δ 1.7-1.9 (m, 3H), 2.2-2.3 (bs, 1H), 2.92 (dd, 1H, J=16 Hz, J=6 Hz), 3.02 (dd, 1H, J=16 Hz, J=6 Hz), 3.15-3.25 (m, 1H), 3.65 (m, 1H), 3.69 (s, 3H), 4.05-4.15 (m, 2H), 5.4-5.5 (m, 1H), 6.9-7.0 (m, 2H), 7.4-7.6 (m, 6H), 7.60 (t, 2H, J= 2 Hz), 7.72 (d, 2H, J=1 Hz), 7.75 (m, 1H), 7.90 (m, 1H).

Step C. N-(3,5-Dichlorobenzenesulfonyl)-2(S)-prolyl-3(R)-amino-3-(4-(2'-cyanophenyl)phenyl)propionic acid

The product described in Step B was hydrolysed to the free acid by adding 2 equivalents of KOH in 3/1 ethanol/water. The solution was acidified with 2.5 N HCl and extracted with methylene chloride. The product was flash chromatographed using 97/3/0.2 methylene chloride/methanol/acetic acid to provide 7 mg of product (50 % yield).
400 MHz $^1H$ NMR ($CD_3OD$): δ 1.7-1.8 (m, 1H), 1.9-2.05 (m, 3H), 2.8-2.95 (m, 2H), 3.3-3.4 (m,2H), 3.5-3.6 (m, 1H), 4.25 (m, 1H), 5.4-5.5 (m, 1H), 7.5-7.6 (m, 6H), 7.7-7.8 (m,5H), 8.80 (m, 1H), MS: m/e 590 (M +1 + $NH_3$).

EXAMPLE 6

N-(3,5-Dichlorobenzenesulfonyl)-2(S)-prolyl-3(R)-amino-3-(4-(2'-formyl)biphenyl)propionic acid

The procedure described in Example 1 and 2, Step D starting with N-(3,5-dichlorobenzenesulfonyl)-2(S)-prolyl-3(R)-amino-3-(4-trifluoromethanesulfonyloxyphenyl)propionic acid, methyl ester 100 mg, 0.167mmol) and 2-formylbenzene boronic acid (130 mg, 0.20 mmol) was followed to provide 45 mg (47% yield) of the methyl ester of the title compound. The methyl ester was hydrolysed to the free acid by adding 2 equivalents of KOH in 3/1 ethanol/water. The solution was acidified with 2.5 N HCl and extracted with methylene chloride. The product was flash chromatographed using 97/3/0.2 methylene chloride/methanol/acetic acid to provide 7 mg of the title compound (50 % yield).
400 MHz $^1H$ NMR ($CDCl_3$): δ 1.7-1.9 (m, 3H), 2.2-2.3 (bs, 1H), 3.02 (dd, 1H, J=16 Hz, J=6 Hz), 3.10 (dd, 1H, J=16 Hz, J=6 Hz), 3.15-3.25 (m, 1H), 3.65 (m, 1H), 4.1-4.2 (m, 2H), 5.4-5.5 (m, 1H), 7.3-7.5 (m, 5H), 7.6-7.7 (m, 2H), 7.71 (d, 2H, J=2 Hz), 7.99 (dd, 2H, J=14 Hz, J=8 Hz), 9.85 (m, 1H).
MS: m/e 593 (M +1 + $NH_3$).

EXAMPLE 7

N-(3,5-Dichlorobenzenesulfonyl)-2(S)-prolyl-3(R)-amino-3-(4-(2'-dimethylaminomethyl)biphenyl)propionic acid.

N-(3,5-Dichlorobenzenesulfonyl)-2(S)-prolyl-3(R)-amino-3-(4-(2'-formyl)biphenyl)propionic acid, methyl ester (from Example 6 (28 mg, 0.047 mmol) was dissolved in methanol (1 mL). Dimethylamine (118 μl, 0.24 mmol of 2M dimethylamine in methanol) was added to the solution along with sodium cyanoborohydride (4.4 mg, 0.07 mmol). The reaction mixture was stirred overnight at 24° C. No starting aldehyde was seen by TLC. Aqueous workup effected an *in situ* hydrolysis of the methyl ester. The reaction mixture was acidified with 2.5 N HCl and extracted with methylene chloride. The product was flash chromatographed using 97/3/0.2 methylene chloride/methanol/acetic acid to provide

3.3 mg of the title compound (15 % yield).
400 MHz $^1$H NMR ($CDCl_3$): δ 1.7-1.9 (m, 3H), 2.2-2.3 (bs, 1H), 2.45 (bs, 6H), 2.92 (dd, 1H, J=16 Hz, J=6 Hz), 3.08 (dd, 1H, J=16 Hz, J=6 Hz), 3.15-3.25 (m, 1H), 3.65 (m, 1H), 4.16 (d, 1H, J=6 Hz), 4.2-4.3 (m, 2H), 5.4-5.5 (m, 1H), 7.20 (d, 2H, J=6 Hz), 7.30 (m, 1H), 7.42 (d, 2H, J=8 Hz), 7.48 (d, 2H, J=8 Hz), 7.59 (t, 1H, J=2 Hz), 7.71 (d, 2H, J=2 Hz), 7.77 (m, 1H), 8.10 (m, 1H).
MS: m/e 608 (M +1 + $NH_3$).

EXAMPLE 8

N-(3,5-Dichlorobenzenesulfonyl)-2(S)-prolyl-3(R)-amino-3-(4-(2'-hydroxymethyl)biphenyl)propionic acid.

N-(3,5-Dichlorobenzenesulfonyl)-2(S)-prolyl-3(R)-amino-3-(4-(2'-formyl)biphenyl)propionic acid, methyl ester (from Example 6 (16 mg, 0.027 mmol)) was dissolved in ethanol (500 μL). Sodium borohydride (2 mg, 0.054 mmol) was added to the reaction mixture and the solution stirred at 24° C for 1 h. No starting aldehyde was seen by TLC (97/3/0.2 methylene chloride/methanol/acetic acid). The reaction mixture was acidified with 2.5 N HCl and extracted with methylene chloride. The product was flash chromatographed using 97/3/0.2 methylene chloride/methanol/acetic acid to provide 11.5 mg of the title compound (73 % yield).
400 MHz $^1$H NMR ($CDCl_3$): δ 1.7-1.9 (m, 3H), 2.15-2.25 (bs, 1H), 2.45 (bs, 6H), 2.94 (dd, 1H, J=16 Hz, J=6 Hz), 3.04 (dd, 1H, J=16 Hz, J=6 Hz), 3.1-3.2 (m, 1H), 3.5-3.6 (m, 1H),3.5-4.3 (vbs, 1H), 4.14 (d, 1H, J=6 Hz), 4.55 (s, 2H), 5.4-5.5 (m, 1H), 7.1-7.2 (m, 2H), 7.2-7.3 (m, 2H), 7.3-7.4 (m 3H), 7.52 (d, 2H, J=8 Hz), 7.59 (t, 1H, J=2 Hz), 7.71 (d, 2H, J=2 Hz), 7.82 (m, 1H).
MS: m/e 595 (M +1 + $NH_3$).

The following compounds were prepared by the procedures described in Example 1 and 2 using the appropriate aryl-halide

| Ex. No | Compound Name | MS* |
|---|---|---|
| 9 | N-(benzenesulfonyl)-2(S)-prolyl-3(R)-amino-3-(4-(2'-methoxyphenyl)phenyl)-propionic acid | 525 (M + $NH_4$) |
| 10 | N-(benzenesulfonyl)-2(S)-methylprolyl-3(R)-amino-3-(4-(2'-methoxyphenyl)phenyl)-propionic acid | 539 (M + $NH_4$) |

EXAMPLE 11

N-(3,5-Dichlorobenzenesulfonyl)-2-methyl-2(S)-prolyl-3(R)-amino-3-(4-(2'-methoxyphenyl)phenyl)propionic acid

Step A. N-tert-Butoxycarbonyl-3(R)-amino-3-(4-hydroxyphenyl)propionic acid, methyl ester.

To a 100 mL round bottom flask fitted with a magnetic stir bar was added 15 mL of water and 30 mL of dioxane. The flask was cooled to 0° and then 3(R)-amino-3-(4-hydroxphenyl)propionic acid, methyl ester, acetic acid salt (3.8 g, 15 mmol), [from Preparative Example 1, Step D] diisopropylethyl amine (DIPEA) (3.5 mL, 30 mmol) and BOC-ON (4.24 g, 17.3 mmole) were added sequentially to the flask. The reaction mixture was stirred for 3 h at 0-5°. The reaction was poured into 100 mL of cold 0.25 N HCl and the mixture was extracted with 5 times 50 mL of ether. Flash chromatography (90/10 hexane/ethyl acetate) removed the forerun by-product and subsequent 70/30 hexane/ethyl acetate eluted the Boc protected amino acid (4.45 g, 80% yield.
400 MHz $^1$H NMR ($CDCl_3$): δ 1.42 (s, 9H), 2.7-2.9 (m, 2H), 3.6 (s, 3H), 5.0 (bs, 1H), 5.4 (bs, 1H), 5.6 (bs, 1H), 6.7 (d, 2H, J=9 Hz), 7.17 (d, 2H, J=9 Hz).

Step B. N-tert-Butoxycarbonyl-3(R)-amino-3-(4-trifluoromethylsulfonyloxyphenyl)propionic acid, methyl ester.

The procedure described in Example 1, Step C was followed using 3.50 g of N-tert-butoxycarbonyl-3(R)-amino-3-(4-hydroxphenyl)propionic acid, methyl ester to provide 4.8 g of desired triflate (90% yield).
400 MHz $^1$H NMR ($CDCl_3$): δ 1.40 (s, 9H), 2.85 (bs, 2H), 3.60 (s, 3H), 5.1 (bs, 1H), 5.60 (bs, 1H), 7.21 (d, 2H, J=8 Hz), 7.36 (d, 2H, J=8 Hz).

Step C. N-tert-Butoxycarbonyl-3(R)-amino-3-(4-(2'-methoxyphenyl)phenyl)propionic acid, methyl ester.

Coupling of 2-methoxybenzeneboronic acid (91 mg, 0.6 mmol) with N-tert-butoxycarbonyl-3(R)-amino-3-(4-trifluoromethylsulfonyloxyphenyl)propionic acid, methyl ester (214 mg, 0.5 mmol) as described in Example 1 and 2, Step D gave 170 mg of the desired product (quantitative yield).
400 MHz $^1$H NMR ($CDCl_3$): δ 1.41 (s, 9H), 2.8-2.9 (bs, 2H), 3.63 (s, 3H), 3.79 (s, 3H), 5.1 (bs, 1H), 5.40 (bs, 1H), 6.9-7.02 (m, 2H), 7.2-7.4 (m, 4H), 7.30 (d, 2H, J=7 Hz), 7.48 (d, 2H, J=7 Hz).

Step D. 3(R)-amino-3-(4-(2'-methoxyphenyl)phenyl)propionic acid, methyl ester hydrochloride.

The title compound was synthesized by the procedure described in Example 1 and 2, Step F by deprotecting the Boc protected amino acid of Step C with anhydrous HCl in ethyl acetate to provide 120 mg of the HCl salt from 170 mg of starting material.

Step E. N-(3,5-Dichlorobenzenesulfonyl)-2-methyl-2(S)-prolyl-3(R)-amino-3-(4-(2'-methoxyphenyl)phenyl)propionic acid, methyl ester.

To a 5 mL round bottom flask fitted with a stir bar and septum was added 3(R)-amino-3-(4-(2'-methoxyphenyl) phenyl)propionic acid, methyl ester hydrochloride (32 mg, 0.1 mmol), diisopropylethyl amine (DIPEA) (72 μl, 0.4 mmol), benzotriazol-1-yloxytripyrrolidino phosphonium hexafluorophosphate (PyBOP) (64 mg, 0.12 mmol) and N-(3,5-dichlorobenzenesulfonyl)-2-methyl-2(S)-proline (36 mg, 0.11 mmol) in 1 ml of methylene chloride. The mixture was stirred overnight at 24° C and worked up by adding 0.5 N HCl (pH=3) and extracting out the product with methylene chloride. The solvent was removed and the residue flash chromatographed (70/30 hexane/ethyl acetate to give the desired product. 400 MHz $^1$H NMR ($CDCl_3$): δ 1.7 (s, 3H), 1.8-1.9 (bs, 1h), 1.92-2.0 (bs, 1H), 2.45-2.55 (bs, 1H), 3.0-3.1 (m, 2H), 3.4-3.5 (m, 1H), 3.73 (s, 3H), 3.78-3.83 (m, 1H), 3.88 (s, 3H), 5.45-5.53 (m, 1H), 7.10-7.2 (m, 2H), 7.3-7.5 (m, 3H), 7.65-7.73 (m, 3H), 7.67 (d, 1H, J=2Hz), 7.70 (d, 2H, J=2 Hz).

Step F. N-(3,5-Dichlorobenzenesulfonyl)-2-methyl-2(S)-prolyl-3(R)-amino-3-(4-(2'-methoxyphenyl)phenyl)propionic acid.

The ester in Step E was hydrolysed to the free acid by adding to 2 equivalents of NaOH in 3/1 ethanol/ water at room temperature. When the hydrolysis was complete, the solvent was removed under reduced pressure and the residue was acidified with 2.5 N HCl. The product was extracted with methylene chloride and chromatographed with (98/1.8/0.2) $CH_2$ $Cl_2$/MeOH/AcOH) to provide 40 mg of the title compound.
400 MHz $^1$H NMR ($CDCl_3$): δ 1.7 (s, 3H), 1.8-1.9 (bs, 1H), 1.92-2.0 (bs, 1H), 2.45-2.55 (bs, 1H), 3.0-3.1 (m, 2H), 3.4-3.5 (m, 1H), 3.78-3.83 (m, 1H), 3.88 (s, 3H), 5.45-5.53 (m, 1H), 7.10-7.2 (m, 2H), 7.3-7.5 (m, 3H), 7.65-7.73 (m, 3H), 7.67 (d, 1H, J=2Hz), 7.70 (d, 2H, J=2 Hz).
MS: m/e 608 (M + $NH_4$).

The following compounds were prepared by the procedures described in Example 11 by coupling the appropriate aryl boronic acid to N-tert-butoxycarbonyl-3(R)-amino-3-(4-trifluoromethylsulfonyloxy phenyl)-propionic acid, methyl ester Example 11, Step B. The aryl boronic acids were synthesized as taught by Galada et al, *Synthesis*-Stuttgart (5),614-(1996), from the corresponding aryl bromide or iodide by transmetallation with t-butyllithium in THF at -78°, followed by treatment with a trialkoxyboronate then subsequent hydrolysis with 2.5 N aqueous HCl. After Boc-deprotection (Example 11, Step D), the resultant β-aminoacid hydrochloride was coupled to either N-(3,5-dichlorobenzenesulfonyl)-2-methyl-2(S)-proline or N-(3,5-dichlorobenzene-sulfonyl)-2(S)-proline by the method taught in Example 11, Step E.

For the compound of Example 28, the starting material N-(3-chlorobenzenesulfonyl)-2-methyl-2(S)-proline was synthesized by the procedure taught in Example 1, Step G using 3-chlorobenzenesulfonyl chloride instead of 3,5-dichlorobenzenesulfonyl chloride.

| Ex. No | Compound Name | MS* |
|---|---|---|
| 12 | N-(benzenesulfonyl)-2-methyl-2(S)-prolyl-3(R)- amino(4-(4'-fluorophenyl)phenyl)-propionic acid | 527 (M + $NH_4$) |
| 13 | N-(3,5-dichlorobenzenesulfonyl)-2(S)-prolyl-3(R)-amino(4-(4'-fluorophenyl)phenyl)-propionic acid | 582 (M + $NH_4$) |
| 14 | N-(3,5-dichlorobenzenesulfonyl)-2(S)-prolyl-3(R)-amino(4-(2'-trifluoromethoxyphenyl) phenyl)-propionic acid | 631 (M + 1) |
| 15 | N-(3,5-dichlorobenzenesulfonyl)-2-methyl-2(S)-prolyl-3(R)-amino(4-(2'-trifluoromethoxyphenyl)-phenyl)propionic acid | 645 (M + 1) |

(continued)

| Ex. No | Compound Name | MS* |
|---|---|---|
| 16 | N-(3,5-dichlorobenzenesulfonyl)-2-methyl-2(S)-prolyl-3(R)-amino-3-(4-(3'-methoxyphenyl)-phenyl)propionic acid | 608 (M + $NH_4$) |
| 17 | N-(3,5-dichlorobenzenesulfonyl)-2(S)-prolyl-3(R)-amino-3-(4-(3'-methoxyphenyl)phenyl) propionic acid | 594 (M + $NH_4$) |
| 18 | N-(3,5-dichlorobenzenesulfonyl)-2-methyl-2(S)-prolyl-3(R)-amino-3-(4-(2'-methoxy-3'-fluorophenyl)phenyl)propionic acid | 626 (M + $NH_4$) |
| 19 | N-(3,5-dichlorobenzenesulfonyl)-2(S)-prolyl-3(R)-amino-3-(4-(2'-methoxy-3'-fluorophenyl) phenyl)-propionic acid | 612 (M + $NH_4$) |
| 20 | N-(3,5-dichlorobenzenesulfonyl)-2-methyl-2(S)-prolyl-3(R)-amino-3-(4-(2'-fluoro-3'-methoxyphenyl)phenyl)propionic acid | 626 (M + $NH_4$) |
| 21 | N-(3,5-dichlorobenzenesulfonyl)-2(S)-prolyl-3(R)-amino-3-(4-(2'-fluoro-3'-methoxyphenyl)-phenyl)propionic acid | 612 (M + $NH_4$) |
| 22 | N-(3,5-dichlorobenzenesulfonyl)-2-methyl-2(S)-prolyl-3(R)-amino-3-(4-(2'-methoxy-5'-fluorophenyl)phenyl)propionic acid | 626 (M + $NH_4$) |
| 23 | N-(3,5-dichlorobenzenesulfonyl)-2(S)-prolyl-3(R)-amino-3-(4-(2'-methoxy-5'-fluorophenyl) phenyl)-propionic acid | 612 (M + $NH_4$) |
| 24 | N-(3,5-dichlorobenzenesulfonyl)-2-methyl-2(S)-prolyl-3(R)-amino-3-(4-(3'-methoxy-5'-fluorophenyl)phenyl)propionic acid | 626 (M + $NH_4$) |
| 25 | N-(3,5-dichlorobenzenesulfonyl)-2(S)-prolyl-3(R)-amino-3-(4-(3'-methoxy-5'-fluorophenyl)-phenyl)propionic acid | 612 (M + $NH_4$) |
| 26 | N-(3,5-dichlorobenzenesulfonyl)-2-methyl-2(S)-prolyl-3(R)-amino-3-(4-(2'-methoxy-6'-fluorophenyl)phenyl)propionic acid | 626 (M + $NH_4$) |
| 27 | N-(3,5-dichlorobenzenesulfonyl)-2(S)-prolyl-3(R)-amino-3-(4-(2'-methoxy-6'-fluorophenyl) phenyl)-propionic acid | 612 (M + $NH_4$) |
| 28 | N-(3-chlorobenzenesulfonyl)-2-methyl-2-(S)-prolyl-3(R)-amino-3-(4-(2'-methoxyphenyl)-phenyl)propionic acid | 574 (M + $NH_4$) |

The following compounds were synthesized by reacting either N-(3,5-dichlorobenzenesulfonyl)-2(S)-prolyl-3 (R)-amino-3-(4-hydroxyphenyl)propionic acid,methyl ester or N-(3,5-dichlorobenzene-sulfonyl)-2-methyl-2(S)-prolyl-3 (R)-amino-3-(4-hydroxyphenyl)propionic acid,methyl ester (as prepared in Preparative Example 1, Step F) with triflic anyhydride according to the procedure described in Example 1 and 2, Step C to form N-(3,5-dichlorobenzenesulfonyl)-2(S)-prolyl-3(R)-amino-3-(4-trifluoromethylsulfonyloxyphenyl)propionic acid, methyl ester or N-(3,5-dichlorobenzenesulfonyl)-2-methyl-2(S)-prolyl-3(R)-amino-3-(4-trifluoromethylsulfonyloxyphenyl)propionic acid, methyl ester; the triflic derivatives were coupled with the appropriate arylboronic acid according to the procedure in Examples 1 and 2, Step D, and the resultant products subsequently hydrolyzed to the free carboxylic acid as described in Example 11, Step F.

| Ex No | Name | MS* |
|---|---|---|
| 29 | N-(3,5-dichlorobenzenesulfonyl)-2-methyl-2(S)-prolyl-3(R)-amino-3-(4-(2'-trifluoromethoxy-4'- fluorophenyl)phenyl)propionic acid | 663 (M + 1) |
| 30 | N-(3,5-dichlorobenzenesulfonyl)-2(S)-prolyl-3(R)-amino-3-(4-(2'-trifluoromethoxy-4'-fluorophenyl)-phenyl)propionic acid | 649 (M + 1) |
| 31 | N-(3,5-dichlorobenzenesulfonyl)-2(S)-prolyl-3(R)-amino-3-(4-(2'-methoxy-4'-fluorophenyl) phenyl)-propionic acid | 612 (M + $NH_4$) |
| 32 | N-(3,5-dichlorobenzenesulfonyl)-2-methyl-2(S)-prolyl-3(R)-amino-3-(4-(2'-methoxy-4'-fluorophenyl)phenyl)propionic acid | 626 (M + $NH_4$) |

EXAMPLE 33

N-(3,5-Dichlorobenzenesulfonyl)-2(S)-prolyl-3(R)-amino-3-(3-(2'-methoxyphenyl)phenyl)propionic acid.

The title compound was prepared by the methods described in Example 3 and 4 except 3(R)-amino-3-(3-hydroxyphenyl)propionic acid, methyl ester was substituted for the 4-hydroxyphenyl derivative.
MS: m/e 594 (M + $NH_4$).

EXAMPLE 34

N-(3,5-Dichlorobenzenesulfonyl)-2(S)-prolyl-3(R)-amino-3-(4-(2'-cyclopropyloxy)biphenyl)propionic acid.

The title compound was prepared by the method described for Preparative Example 1. The 2-cyclopropyloxyphenylboronic acid was prepared by the method of V. Snieckus et al. (J. Org. Chem. **1991**, *56*, 3763) from 1-bromo-2-cyclopropyloxybenzene (Petinskii, A. A. et al. Bull. Acad. Sci. USSR Div. Chem. Sci. (Engl. Transl.) **1972**, *21* 1720) via lithium halogen. The final product (34 mg) was obtained after NaOH hydrolysis of the ester.
500 MHz $^1$H NMR ($CD_3OD$): δ 0.60-0.66 (m, 2H), 0.70-0.76 (m, 2H), 1.7-1.8 (m, 1H), 1.95-2.05 (m, 3H), 2.90-2.95 (m, 2H), 3.3-3.4 (m, 1H), 3.5-3.6 (m, 1H), 3.76 (tt, J=6.0, 3.0 Hz, 1H), 4.22-4.30 (m, 1H), 5.36 (dd, J = 7.0, 7.0 Hz, 1H), 7.00 (td, J= 7.5, 1.0 Hz, 1H), 7.26 (dd, J=7.5, 1.5 Hz, 1H), 7.29 (ddd, J= 7.5, 7.5. 1.5 Hz,1H), 7.36-7.46 (m, 5H), 7.73 (t, J=1.5 Hz, 1H), 7.78 (d, J=1.5 Hz. 2H).
MS: m/e 620 (M + $NH_4$).

EXAMPLE 35

N-(3,5-Dichlorobenzenesulfonyl)-2-methyl-2(S)-prolyl-3(R)-amino-3-(4-(2'-cyclopropyloxy)biphenyl)propionic acid

The title compound was prepared by the methods described in Examples 11 and 34 substituting N-(3,5-dichlorobenzenesulfonyl)-2-methyl-2(S)-proline for N-(3,5-dichlorobenzenesulfonyl)-2(S)-proline in the coupling reaction. The final product (82 mg) was obtained after NaOH hydrolysis of the ester.
500 MHz $^1$H NMR ($CD_3OD$): δ 0.58-0.64 (m, 2H), 0.70-0.76 (m, 2H), 1.67 (s, 1H), 1.86-2.00 (m, 3H), 2.22-2.28 (m, 1H), 2.88-3.00 (m, 2H), 3.44-3.52 (m, 1H), 3.54-3.60 (m, 1H), 3.75 (tt, J=6.0, 3.0 Hz, 1H), 5.34-5.40 (m, 1H), 6.99 (td, J= 7.0, 1.5 Hz, 1H), 7.25 (dd, J=7.5, 2.0 Hz, 1H), 7.29 (ddd, J= 7.5, 7.0. 1.5 Hz,1H), 7.37 (dd, J=7.5, 1.5 Hz, 1H), 7.39-7.44 (m, 4H), 7.68-7.72 (m, 3H).
MS: m/e 634 (M + $NH_4$).

EXAMPLE 36

Inhibition of VLA-4 Dependent Adhesion to BSA-CS-1 Conjugate

Step A. Preparation of CS-1 Coated Plates.

Untreated 96 well polystyrene flat bottom plates were coated with bovine serum albumin (BSA; 20 μg/ml) for 2 hours at room temperature and washed twice with phosphate buffered saline (PBS). The albumin coating was next derivatized with 10 μg/ml 3-(2-pyridyldithio) propionic acid N-hydroxysuccinimide ester (SPDP), a heterobifunctional crosslinker, for 30 minutes at room temperature and washed twice with PBS. The CS-1 peptide (Cys-Leu-His-Gly-Pro-Glu-Ile-Leu-Asp-Val-Pro-Ser-Thr), which was synthesized by conventional solid phase chemistry and purified by reverse phase HPLC, was next added to the derivatized BSA at a concentration of 2.5 μg/ml and allowed to react for 2 hours at room temperature. The plates were washed twice with PBS and stored at 4°C.

Step B. Preparation of Fluorescently Labeled Jurkat Cells.

Jurkat cells, clone E6-1, obtained from the American Type Culture Collection (Rockville, MD; cat # ATCC TIB-152) were grown and maintained in RPMI-1640 culture medium containing 10% fetal calf serum (FCS), 50 units/ml penicillin, 50 μg/ml streptomycin and 2 mM glutamine. Fluorescence activated cell sorter analysis with specific monoclonal antibodies confirmed that the cells expressed both the α4 and β1 chains of VLA-4. The cells were centrifuged at 400xg for five minutes and washed twice with PBS. The cells were incubated at a concentration of 2 x $10^6$ cells/ml in PBS containing a 1 μM concentration of a fluorogenic esterase substrate (2', 7'-bis-(2-carboxyethyl)-5-(and -6)-carboxyfluorescein, acetoxymethyl ester; BCECF-AM; Molecular Probes Inc., Eugene, Oregon; catalog #B-1150) for 30-60 minutes at 37°C in a 5% $CO_2$/air incubator. The fluorescently labeled Jurkat cells were washed two times in PBS and resuspended in RPMI containing 0.25% BSA at a final concentration of 2.0 x $10^6$ cells/ml.

Step C. Assay Procedure.

Compounds of this invention were prepared in DMSO at 100x the desired final assay concentration. Final concentrations were selected from a range between 0.001 nM-100 μM. Three μL of diluted compound, or vehicle alone, were premixed with 300 μL of cell suspension in 96-well polystyrene plates with round bottom wells. 100 μL aliquots of the cell /compound mixture were then transferred in duplicate to CS-1 coated wells. The cells were next incubated for 30 minutes at room temperature. The non-adherent cells were removed by two gentle washings with PBS. The remaining adherent cells were quantitated by reading the plates on a Cytofluor II fluorescence plate reader (Perseptive Biosystems Inc., Framingham, MA; excitation and emission filter settings were 485 nm and 530 nm, respectively). Control wells containing vehicle alone were used to determine the level of cell adhesion corresponding to 0% inhibition. Control wells coated with BSA and crosslinker (no CS-1 peptide) were used to determine the level of cell adhesion corresponding to 100% inhibition. Cell adhesion to wells coated with BSA and crosslinker was usually less than 5% of that observed to CS-1 coated wells in the presence of vehicle. Percent inhibition was then calculated for each test well and the $IC_{50}$ was determined from a ten point titration using a validated four parameter fit algorithm.

EXAMPLE 37

Antagonism of VLA-4 Dependent Binding to VCAM-Ig Fusion Protein.

Step A. Preparation of VCAM-Ig.

The signal peptide as well as domains 1 and 2 of human VCAM (GenBank Accession no. M30257) were amplified by PCR using the human VCAM cDNA (R & D Systems) as template and the following primer sequences:
3'-PCR primer:5'-AATTATAATTTGATCAACTTAC
CTGTCAATTCTTTTACAGCCTGCC-3';
5'-PCR primer:
5'-ATAGGAATTCCAGCTGCCACCATGCCTGGGAAGATGGTCG-3'.

The 5'-PCR primer contained EcoRI and PvuII restriction sites followed by a Kozak consensus sequence (CCACC) proximal to the initiator methionine ATG. The 3'-PCR primer contained a BclI site and a splice donor sequence. PCR was performed for 30 cycles using the following parameters: 1 min. at 94°C, 2 min. at 55°C, and 2 min. at 72°C. The amplified region encoded the following sequence of human VCAM-1: MPGKMVVILGASNILWIMFAASQAFKIET-TPESRYLAQIGDSVSLTCST TGCESPFFSWRTQIDSPLNGKVTNEGTTSTLTMNPVSFGNEHSYLCTA TCESRKLE KGIQVEIYSFPKDPEIHLSGPLEAGKPITVKCSVADVYPFD RLEIDLLKGDHLMKSQEFLEDADRKSLETKSLEVTFT-PVIEDIGKVLVC RAKLHIDEMDSVPTVRQAVKEL. The resulting PCR product of 650 bp was digested with EcoRI and BclI and ligated to expression vector pIg-Tail (R & D Systems, Minneapolis, MN) digested with EcoRI and BamHI. The pIg-Tail vector contains the genomic fragment which encodes the hinge region, CH2 and CH3 of human IgG1 (GenBank Accession no. Z17370). The DNA sequence of the resulting VCAM fragment was verified using Sequenase (US Biochemical, Cleveland, OH). The fragment encoding the entire VCAM-Ig fusion was subsequently excised from pIg-Tail with EcoRI and NotI and ligated to pCI-neo (Promega, Madison, WI) digested with EcoRI and NotI. The resulting vector, designated pCI-neo/VCAM-Ig was transfected into CHO-K1 (ATCC CCL 61) cells using calcium-phosphate DNA precipitation (Specialty Media, Lavalette, NJ). Stable VCAM-Ig producing clones were selected according to standard protocols using 0.2-0.8 mg/ml active G418 (Gibco, Grand Island, NY), expanded, and cell supernatants were screened for their ability to mediate Jurkat adhesion to wells previously coated with 1.5 μg/ml (total protein) goat anti-human IgG (Sigma, St. Louis, MO). A positive CHO-K1/VCAM-Ig clone was subsequently adapted to CHO-SFM serum-free media (Gibco) and maintained under selection for stable expression of VCAM-Ig. VCAM-Ig was purified from crude culture supernatants by affinity chromatography on Protein A/G Sepharose (Pierce, Rockford, IL) according to the manufacturer's instructions and desalted into 50 mM sodium phosphate buffer, pH 7.6, by ultrafiltration on a YM-30 membrane (Amicon, Beverly, MA).

Step B. Preparation of $^{125}$I-VCAM-Ig.

VCAM-Ig was labeled to a specific radioactivity greater that 1000 Ci/mmole with $^{125}$I-Bolton Hunter reagent (New England Nuclear, Boston, MA; cat # NEX120-0142) according to the manufacturer's instructions.The labeled protein was separated from unincorporated isotope by means of a calibrated HPLC gel filtration column (G2000SW; 7.5 x 600 mm; Tosoh, Japan) using uv and radiometric detection.

Step C. VCAM-Ig Binding Assay.

Compounds of this invention were prepared in DMSO at 100x the desired final assay concentration. Final concentrations were selected from a range between 0.001 nM-100 μM. Jurkat cells were centrifuged at 400xg for five minutes and resuspended in binding buffer (25 mM HEPES, 150 mM NaCl, 3 mM KCl, 2 mM glucose, 0.1% bovine serum albumin, pH 7.4). The cells were centrifuged again and resuspended in binding buffer supplemented with $MnCl_2$ at a final concentration of 1 mM. Compounds were assayed in Millipore MHVB multiscreen plates (cat# MHVBN4550, Millipore Corp., MA) by making the following additions to duplicate wells: (i) 200 μL of binding buffer containing 1 mM $MnCl_2$; (ii) 20 μL of $^{125}$I-VCAM-Ig in binding buffer containing 1 mM $MnCl_2$ (final assay concentration ~ 100 pM); (iii) 2.5 μL of compound solution or DMSO; (iv) and 0.5 x $10^6$ cells in a volume of 30 μL. The plates were incubated at room temperature for 30 minutes, filtered on a vacuum box, and washed on the same apparatus by the addition of 100 μL of binding buffer containing 1 mM $MnCl_2$. After insertion of the multiscreen plates into adapter plates (Packard, Meriden, CT, cat# 6005178), 100 μL of Microscint-20 (Packard cat# 6013621) was added to each well. The plates were then sealed, placed on a shaker for 30 seconds, and counted on a Topcount microplate scintillation counter (Packard). Control wells containing DMSO alone were used to determine the level of VCAM-Ig binding corresponding to 0% inhibition. Contol wells in which cells were omitted were used to determine the level of binding corresponding to 100% inhibition. Binding of $^{125}$I-VCAM-Ig in the absence of cells was usually less than 5% of that observed using cells in the presence of vehicle. Percent inhibition was then calculated for each test well and the $IC_{50}$ was determined from a ten point titration using a validated four parameter fit algorithm.

EXAMPLE 38

Antagonism of $\alpha_4\beta_7$ Dependent Binding to VCAM-Ig Fusion Protein.

Step A. $\alpha_4\beta_7$ Cell line.

RPMI-8866 cells (a human B cell line $\alpha_4^+\beta_1^-\beta_7^+$; a gift from Prof. John Wilkins, University of Manitoba, Canada) were grown in RPMI/10% fetal calf serum/ 100 U penicillin/100 μg streptomycin/2 mM L-glutamine at 37°C, 5 % carbon dioxide. The cells were pelleted at 1000 rpm for 5 minutes and then washed twice and resuspended in binding buffer (25 mM Hepes, 150 mM NaCl , 0.1 % BSA, 3 mM KCl, 2 mM Glucose, pH 7.4).

Step B. VCAM-Ig Binding Assay.

Compounds of this invention were prepared in DMSO at 100x the desired final assay concentration. Final concentrations were selected from a range between 0.001 nM-100 μM. Compounds were assayed in Millipore MHVB multiscreen plates (Cat# MHVBN4550) by making the following sequential additions to duplicate wells: (i) 100 μl/well of binding buffer containing 1.5 mM $MnCl_2$; (ii) 10 μl/well $^{125}$I-VCAM-Ig in binding buffer (final assay concentration < 500 pM); (iii) 1.5 μl/well test compound or DMSO alone; (iv) 38 μl/well RPMI-8866 cell suspension (1.25 x $10^6$ cells/well). The plates were incubated at room temperature for 45 minutes on a plate shaker at 200 rpm, filtered on a vacuum box, and washed on the same apparatus by the addition of 100 μL of binding buffer containing 1 mM $MnCl_2$. After insertion of the multiscreen plates into adapter plates (Packard, Meriden, CT, cat# 6005178), 100 μL of Microscint-20 (Packard cat# 6013621) was added to each well. The plates were then sealed, placed on a shaker for 30 seconds, and counted on a Topcount microplate scintillation counter (Packard). Control wells containing DMSO alone were used to determine the level of VCAM-Ig binding corresponding to 0% inhibition. Wells in which cells were omitted were used to determine the level of binding corresponding to 100% inhibition. Percent inhibition was then calculated for each test well and the $IC_{50}$ was determined from a ten point titration using a validated four parameter fit algorithm.

## Claims

1. A compound having the formula I:

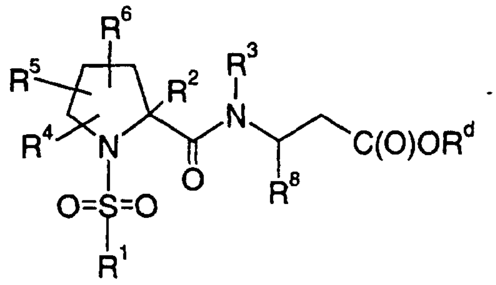

I

or a pharmaceutically acceptable salt thereof wherein:

$R^1$ is phenyl optionally substituted with one to four substituents independently selected from $R^b$;
$R^2$ is hydrogen or $C_{1-6}$alkyl;
$R^3$ is

1) hydrogen,
2) $C_{1-10}$ alkyl,
3) Cy, or
4) Cy-$C_{1-10}$ alkyl,

wherein alkyl is optionally substituted with one to four substituents independently selected from $R^a$; and Cy is optionally substituted with one to four substituents independently selected from $R^b$;
$R^4$, $R^5$ and $R^6$ are each independently selected from the group consisting of

1) hydrogen, or
2) a group selected from $R^b$;

$R^8$ is Cy-($Cy^1$) wherein Cy and $Cy^1$ are each phenyl optionally substituted with one to four substituents independently selected from $R^b$;
$R^a$ is

1) $-CF_3$,
2) $-OR^d$,
3) $-NO_2$,
4) halogen
5) $-S(O)_mR^d$,
6) $-SR^d$,
7) $-S(O)_2OR^d$,
8) $-S(O)_mNR^dR^e$,
9) $-NR^dR^e$,
10) $-O(CR^fR^g)_nNR^dR^e$,
11) $-C(O)R^d$,
12) $-CO_2R^d$,
13) $-CO_2(CR^fR^g)_nCONR^dR^e$,
14) $-OC(O)R^d$,
15) -CN,
16) $-C(O)NR^dR^e$,
17) $-NR^dC(O)R^e$,
18) $-OC(O)NR^dR^e$,
19) $-NR^dC(O)OR^e$,
20) $-NR^dC(O)Nr^dR^e$,
21) $-CR^d(N-OR^e)$, or
22) Cy optionally substituted with a group independently

selected from $R^c$;
$R^b$ is

1) a group selected from $R^a$,
2) $C_{1-10}$ alkyl,
3) $C_{2-10}$ alkenyl,
4) $C_{2-10}$ alkynyl, or
5) Cy-$C_{1-10}$ alkyl,

wherein alkyl, alkenyl, alkynyl, and Cy are optionally substituted with a group independently selected from $R^c$;
$R^c$ is

1) halogen,
2) CN,
3) NH($C_{1-5}$alkyl),
4) N($C_{1-5}$alkyl)$_2$,
5) amino,
6) carboxy,
7) $C_{1-4}$alkyl,
8) $C_{1-4}$alkoxy,
9) aryl,
10) aryl $C_{1-4}$alkyl, or
11) aryloxy;

$R^d$ and $R^e$ are independently selected from hydrogen, $C_{1-10}$alkyl, $C_{2-10}$alkenyl, $C_{2-10}$alkynyl, Cy and Cy-$C_{1-10}$alkyl, wherein alkyl, alkenyl, alkynyl and Cy is optionally substituted with one to four substituents independently selected from $R^c$; or
$R^d$ and $R^e$ together with the atoms to which they are attached form a heterocyclic ring of 5 to 7 members containing 0-2 additional heteroatoms independently selected from oxygen, sulfur and nitrogen;
$R^f$ and $R^g$ are independently selected from hydrogen, $C_{1-10}$alkyl, Cy and Cy-$C_{1-10}$alkyl; or
$R^f$ and $R^g$ together with the carbon to which they are attached form a ring of 5 to 7 members containing 0-2 heteroatoms independently selected from oxygen, sulfur and nitrogen;
Cy and $Cy^1$are

1) cycloalkyl,
2) heterocyclyl,
3) aryl, or
4) heteroaryl;

m is an integer from 1 to 2; and
n is an integer from 1 to 10.

**2.** A compound of Claim 1 wherein $R^1$ is phenyl optionally substituted with one to two groups independently selected from halogen, O-$C_{1-3}$alkyl, and trifluoromethyl.

**3.** A compound of Claim 1 or Claim 2 wherein $R^1$ is 3,5-dichlorophenyl or 3-trifluoromethylphenyl.

**4.** A compound of any one of Claims 1 to 3 wherein $R^8$ is optionally substituted biphenyl, wherein the optional substituents are one or two groups independently selected from halogen, CN, $OR^d$, O(CO)$R^d$, $C_{1-5}$alkyl optionally substituted with one or two groups selected from $R^c$, $CF_3$, and OC(O)NRdRe.

**5.** A compound of Claim 1 having the formula Ib:

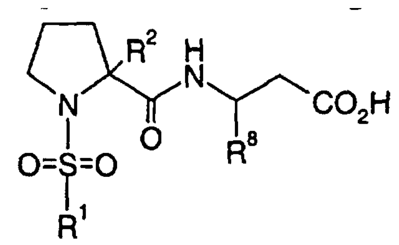

Ib

wherein
$R^1$ is phenyl optionally substituted with one to four substituents independently selected from $R^b$;
$R^2$ is H or $C_{1-6}$ alkyl; and
$R^8$ is Cy-($Cy^1$), wherein Cy and $Cy^1$ are each phenyl optionally substituted with one to four substituents independently selected from $R^b$.

**6.** A compound of Claim 5 wherein
$R^1$ is phenyl optionally substituted with one or two groups selected from halogen, O-$C_{1-3}$alkyl, and trifluoromethyl;
$R^2$ is H or methyl;
$R^8$ is optionally substituted biphenyl wherein the optional substituents are one or two groups independently selected from halogen, CN, $OR^d$, $O(CO)R^d$, $C_{1-5}$alkyl optionally substituted with one or two groups selected from $R^c$, $CF_3$, and $OC(O)NR^dR^e$.

**7.** A compound as claimed in any one of Claims 1 to 5 selected from the group consisting of:

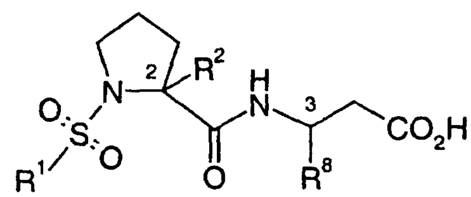

| 2/3* | $R^1$ | $R^2$ | $R^8$ |
|---|---|---|---|
| S/R | 3,5-diCl-Ph | H | biphenyl |
| S/S | 3,5-diCl-Ph | H | biphenyl |
| S/R | 3,5-diCl-Ph | H | 2'-methoxybiphenyl |
| S/S | 3,5-diCl-Ph | H | 2'-methoxybiphenyl |
| S/R | 3,5-diCl-Ph | H | 2'-cyanobiphenyl |
| S/R | 3,5-diCl-Ph | H | 2'-formylbiphenyl |
| S/R | 3,5-diCl-Ph | H | 2'-dimethylaminomethylbiphenyl |
| S/R | 3,5-diCl-Ph | H | 2'-hydroxymethylbiphenyl |
| S/R | Ph | H | 2'-methoxybiphenyl |
| S/R | Ph | $CH_3$ | 2'-methoxybiphenyl |
| S/R | 3,5-diCl-Ph | $CH_3$ | 2'-methoxybiphenyl |
| S/R | Ph | $CH_3$ | 4'-fluorobiphenyl |
| S/R | 3,5-diCl-Ph | H | 4'-fluorobiphenyl |
| S/R | 3,5-diCl-Ph | H | 2'-$CF_3$O-biphenyl |
| S/R | 3,5-diCl-Ph | $CH_3$ | 2'-$CF_3$O-biphenyl |
| S/R | 3,5-diCl-Ph | $CH_3$ | 3'-methoxybiphenyl |
| S/R | 3,5-diCl-Ph | H | 3'-methoxybiphenyl |

*Stereoconfiguration at the indicated positions

(continued)

| 2/3* | $R^1$ | $R^2$ | $R^8$ |
|---|---|---|---|
| S/R | 3,5-diCl-Ph | $CH_3$ | 2'-methoxy-3'-F-biphenyl |
| S/R | 3,5-diCl-Ph | H | 2'-methoxy-3'-F-biphenyl |
| S/R | 3,5-diCl-Ph | $CH_3$ | 3'-methoxy-2'-F-biphenyl |
| S/R | 3,5-diCl-Ph | H | 3'-methoxy-2'-F-biphenyl |
| S/R | 3,5-diCl-Ph | $CH_3$ | 2'-methoxy-5'-F-biphenyl |
| S/R | 3,5-diCl-Ph | H | 2'-methoxy-5'-F-biphenyl |
| S/R | 3,5-diCl-Ph | CH3 | 3'-methoxy-5'-F-biphenyl |
| S/R | 3,5-diCl-Ph | H | 3'-methoxy-5'-F-biphenyl |
| S/R | 3,5-diCl-Ph | $CH_3$ | 2'-methoxy-6'-F-biphenyl |
| S/R | 3,5-diCl-Ph | H | 2'-methoxy-6'-F-biphenyl |
| S/R | 3-Cl-Ph | $CH_3$ | 2'-methoxybiphenyl |
| S/R | 3,5-diCl-Ph | $CH_3$ | 2'-$CF_3O$-4'-F-biphenyl |
| S/R | 3,5-diCl-Ph | H | 2'-$CF_3O$-4'-F-biphenyl |
| S/R | 3,5-diCl-Ph | H | 2'-methoxy-4'-F-biphenyl |
| S/R | 3,5-diCl-Ph | $CH_3$ | 2'-methoxy-4'-F-biphenyl |
| S/R | 3,5-diCl-Ph | H | 3-(2'-methoxyphenyl)-phenyl |
| S/R | 3,5-diCl-Ph | H | 4-(2'-cyclopropoxy)-biphenyl |
| S/R | 3,5-diCl-Ph | $CH_3$ | 4-(2'-cyclopropoxy)-biphenyl |

*Stereoconfiguration at the indicated positions

**8.** The use of a compound as claimed in any one of Claims 1 to 7 for the manufacture of a medicament for inhibiting cell adhesion.

**9.** The use of a compound as claimed in any one of Claims 1 to 7 for the manufacture of a medicament for the treatment of diseases, disorders, conditions or symptoms mediated by cell adhesion.

**10.** The use of a compound as claimed in any one of Claims 1 to 7 for the manufacture of a medicament for the treatment of asthma.

**11.** The use of a compound as claimed in any one of Claims 1 to 7 for the manufacture of a medicament for the treatment of allergic rhinitis.

**12.** The use of a compound as claimed in any one of Claims 1 to 7 for the manufacture of a medicament for the treatment of multiple sclerosis.

**13.** The use of a compound as claimed in any one of Claims 1 to 7 for the manufacture of a medicament for the treatment of atherosclerosis.

**14.** The use of a compound as claimed in any one of Claims 1 to 7 for the manufacture of a medicament for the treatment of inflammation.

**15.** The use of a compound as claimed in any one of Claims 1 to 7 for the manufacture of a medicament for the treatment of inflammatory bowel disease.

**16.** A pharmaceutical composition which comprises a compound as claimed in any one of Claims 1 to 7 and a pharmaceutically acceptable carrier thereof.

## Patentansprüche

**1.** Eine Verbindung mit der Formel I:

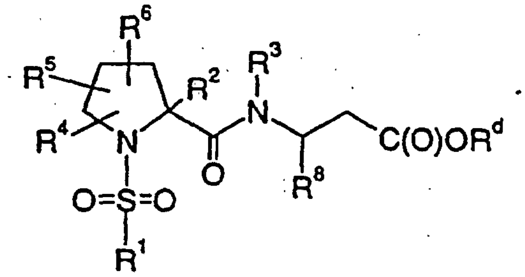

I

oder ein pharmazeutisch annehmbares Salz davon, wobei:

$R^1$ Phenyl ist, gegebenenfalls substituiert mit ein bis vier Substituenten, unabhängig ausgewählt aus $R^b$,
$R^2$ Wasserstoff oder $C_{1-6}$-Alkyl ist,
$R^3$ ist

1) Wasserstoff,
2) $C_{1-10}$-Alkyl,
3) Cy oder
4) Cy-$C_{1-10}$-Alkyl,

wobei Alkyl gegebenenfalls substituiert ist mit ein bis vier Substituenten, unabhängig ausgewählt aus $R^a$, und Cy gegebenenfalls substituiert ist mit ein bis vier Substituenten, unabhängig ausgewählt aus $R^b$,
$R^4$, $R^5$ und $R^6$ jeweils unabhängig ausgewählt sind aus der Gruppe, bestehend aus

1) Wasserstoff oder
2) einer Gruppe, ausgewählt aus $R^b$,

$R^8$ Cy-($Cy^1$) ist, wobei Cy und $Cy^1$ jeweils Phenyl sind, gegebenenfalls substituiert mit ein bis vier Substituenten, unabhängig ausgewählt aus $R^b$,
$R^a$ ist

1) $-CF_3$,
2) $-OR^d$,
3) $-NO_2$,
4) Halogen,
5) $-S(O)_mR^d$,
6 ) $-SR^d$,
7) $-S(O)_2OR^d$,
8) $-S(O)_mNR^dR^e$,
9) $-NR^dR^e$,
10) $-O(CR^fR^g)_nNR^dR^e$,
11) $-C(O)R^d$,
12) $-CO_2R^d$,
13) $-CO_2(CR^fR^g)_nCONR^dR^e$,
14 ) $-OC(O)R^d$,
15) -CN,
16) $-C(O)NR^dR^e$,
17) $-NR^dC(O)R^e$,
18) $-OC(O)NR^dR^e$,
19) $-NR^dC(O)OR^e$,
20) $-NR^dC(O)NR^dR^e$,
21) $-CR^d(N-OR^e)$ oder
22) Cy, gegebenenfalls substituiert mit einer

Gruppe, unabhängig ausgewählt aus $R^c$,
$R^b$ ist

1) eine Gruppe, ausgewählt aus $R^a$,
2) $C_{1-10}$-Alkyl,
3) $C_{2-10}$-Alkenyl,
4) $C_{2-10}$-Alkinyl oder
5) Cy-$C_{1-10}$-Alkyl,

wobei Alkyl, Alkenyl, Alkinyl und Cy gegebenenfalls substituiert sind mit einer Gruppe, unabhängig ausgewählt aus $R^c$,
$R^c$ ist

1) Halogen,
2) CN,
3) NH($C_{1-5}$-Alkyl),
4) N($C_{1-5}$-Alkyl)$_2$,
5) Amino,
6) Carboxy,
7) $C_{1-4}$-Alkyl,
8) $C_{1-4}$-Alkoxy,
9) Aryl ,
10) Aryl-$C_{1-4}$-alkyl oder
11) Aryloxy,

$R^d$ und $R^e$ unabhängig ausgewählt sind aus Wasserstoff, $C_{1-10}$-Alkyl, $C_{2-10}$-Alkenyl, $C_{2-10}$-Alkinyl, Cy und Cy-$C_{1-10}$-Alkyl, wobei Alkyl, Alkenyl, Alkinyl und Cy gegebenenfalls substituiert sind mit ein bis vier Substituenten, unabhängig ausgewählt aus $R^c$, oder
$R^d$ und $R^e$ zusammen mit den Atomen, an die sie gebunden sind, einen heterocyclischen Ring mit 5 bis 7 Gliedern bilden, der 0-2 zusätzliche Heteroatome enthält, unabhängig ausgewählt aus Sauerstoff, Schwefel und Stickstoff,
$R^f$ und $R^g$ unabhängig ausgewählt sind aus Wasserstoff, $C_{1-10}$-Alkyl, Cy und Cy-$C_{1-10}$-Alkyl oder
$R^f$ und $R^g$ zusammen mit dem Kohlenstoff, an den sie gebunden sind, einen Ring mit 5 bis 7 Gliedern bilden, der 0-2 Heteroatome enthält, unabhängig ausgewählt aus Sauerstoff, Schwefel und Stickstoff,
Cy und $Cy^1$ sind

1) Cycloalkyl,
2) Heterocyclyl,
3) Aryl oder
4) Heteroaryl,

m eine ganze Zahl von 1 bis 2 ist und
n eine ganze Zahl von 1 bis 10 ist.

**2.** Eine Verbindung nach Anspruch 1, wobei $R^1$ Phenyl ist, gegebenenfalls substituiert mit ein bis zwei Gruppen, unabhängig ausgewählt aus Halogen, O-$C_{1-3}$-Alkyl und Trifluormethyl.

**3.** Eine Verbindung nach Anspruch 1 oder Anspruch 2, wobei $R^1$ 3,5-Dichlorphenyl oder 3-Trifluormethylphenyl ist.

**4.** Eine Verbindung nach irgendeinem der Ansprüche 1 bis 3, wobei $R^8$ gegebenenfalls substituiertes Biphenyl ist, wobei die optionalen Substituenten ein oder zwei Gruppen sind, unabhängig ausgewählt aus Halogen, CN, $OR^d$, $O(CO)R^d$, $C_{1-5}$-Alkyl, gegebenenfalls substituiert mit ein oder zwei Gruppen, ausgewählt aus $R^c$, $CF_3$ und OC(O)$NR^dR^e$.

**5.** Eine Verbindung nach Anspruch 1 mit der Formel Ib:

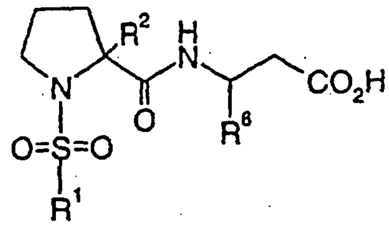

Ib

wobei
$R^1$ Phenyl ist, gegebenenfalls substituiert mit ein bis vier Substituenten, unabhängig ausgewählt aus $R^b$,
$R^2$ H oder $C_{1-6}$-Alkyl ist und
$R^8$ Cy-($Cy^1$) ist, wobei Cy und $Cy^1$ jeweils Phenyl sind, gegebenenfalls substituiert mit ein bis vier Substituenten, unabhängig ausgewählt aus $R^b$.

**6.** Eine Verbindung nach Anspruch 5, wobei
$R^1$ Phenyl ist, gegebenenfalls substituiert mit ein oder zwei Gruppen, ausgewählt aus Halogen, O-$C_{1-3}$-Alkyl und Trifluormethyl,
$R^2$ H oder Methyl ist,
$R^8$ gegebenenfalls substituiertes Biphenyl ist, wobei die optionalen Substituenten ein oder zwei Gruppen sind, unabhängig ausgewählt aus Halogen, CN, $OR^d$, $O(CO)R^d$, $C_{1-5}$-Alkyl, gegebenenfalls substituiert mit ein oder zwei Gruppen, ausgewählt aus $R^c$, $CF_3$ und $OC(O)NR^dR^e$.

**7.** Eine wie in irgendeinem der Ansprüche 1 bis 5 beanspruchte Verbindung, ausgewählt aus der Gruppe, bestehend aus:

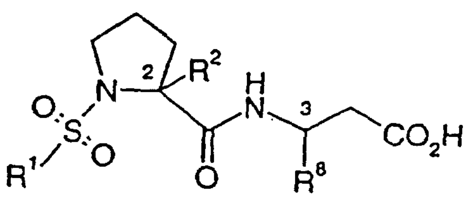

| 2/3* | $R^1$ | $R^2$ | $R^8$ |
|---|---|---|---|
| S/R | 3,5-Di-Cl-Ph | H | Biphenyl |
| S/S | 3,5-Di-Cl-Ph | H | Biphenyl |
| S/R | 3,5-Di-Cl-Ph | H | 2'-Methoxybiphenyl |
| S/S | 3,5-Di-Cl-Ph | H | 2'-Methoxybiphenyl |
| S/R | 3,5-Di-Cl-Ph | H | 2'-Cyanobiphenyl |
| S/R | 3,5-Di-Cl-Ph | H | 2'-Formylbiphenyl |
| S/R | 3,5-Di-Cl-Ph | H | 2'-Dimethylaminomethylbiphenyl |
| S/R | 3,5-Di-Cl-Ph | H | 2'-Hydroxymethylbiphenyl |
| S/R | Ph | H | 2'-Methoxybiphenyl |
| S/R | Ph | $CH_3$ | 2'-Methoxybiphenyl |
| S/R | 3,5-Di-Cl-Ph | $CH_3$ | 2'-Methoxybiphenyl |

* Stereokonfiguration an den angegebenen Positionen

(fortgesetzt)

| 2/3* | $R^1$ | $R^2$ | $R^8$ |
|---|---|---|---|
| S/R | Ph | $CH_3$ | 4'-Fluorbiphenyl |
| S/R | 3,5-Di-Cl-Ph | H | 4'-Fluorbiphenyl |
| S/R | 3,5-Di-Cl-Ph | H | 2'-$CF_3$O-Biphenyl |
| S/R | 3,5-Di-Cl-Ph | $CH_3$ | 2'-$CF_3$O-Biphenyl |
| S/R | 3,5-Di-Cl-Ph | $CH_3$ | 3'-Methoxybiphenyl |
| S/R | 3,5-Di-Cl-Ph | H | 3'-Methoxybiphenyl |
| S/R | 3,5-Di-Cl-Ph | $CH_3$ | 2'-Methoxy-3'-F-biphenyl |
| S/R | 3,5-Di-Cl-Ph | H | 2'-Methoxy-3'-F-biphenyl |
| S/R | 3,5-Di-Cl-Ph | $CH_3$ | 3'-Methoxy-2'-F-biphenyl |
| S/R | 3,5-Di-Cl-Ph | H | 3'-Methoxy-2'-F-biphenyl |
| S/R | 3,5-Di-Cl-Ph | $CH_3$ | 2'-Methoxy-5'-F-biphenyl |
| S/R | 3,5-Di-Cl-Ph | H | 2'-Methoxy-5'-F-biphenyl |
| S/R | 3,5-Di-Cl-Ph | $CH_3$ | 3'-Methoxy-5'-F-biphenyl |
| S/R | 3,5-Di-Cl-Ph | H | 3'-Methoxy-5'-F-biphenyl |
| S/R | 3,5-Di-Cl-Ph | $CH_3$ | 2'-Methoxy-6'-F-biphenyl |
| S/R | 3,5-Di-Cl-Ph | H | 2'-Methoxy-6'-F-biphenyl |
| S/R | 3-Cl-Ph | $CH_3$ | 2'-Methoxybiphenyl |
| S/R | 3,5-Di-Cl-Ph | $CH_3$ | 2'-$CF_3$O-4'-Biphenyl |
| S/R | 3,5-Di-Cl-Ph | H | 2'-$CF_3$O-4'-Biphenyl |
| S/R | 3,5-Di-Cl-Ph | H | 2'-Methoxy-4'-F-biphenyl |
| S/R | 3,5-Di-Cl-Ph | $CH_3$ | 2'-Methoxy-4'-F-biphenyl |
| S/R | 3,5-Di-Cl-Ph | H | 3-(2'-Methoxyphenyl)phenyl |
| S/R | 3,5-Di-Cl-Ph | H | 4-(2'-Cyclopropoxy)biphenyl |
| S/R | 3,5-Di-Cl-Ph | $CH_3$ | 4-(2'-Cyclopropoxy)biphenyl |

* Stereokonfiguration an den angegebenen Positionen

**8.** Die Verwendung einer wie in irgendeinem der Ansprüche 1 bis 7 beanspruchten Verbindung zur Herstellung eines Medikaments zur Inhibierung von Zelladhäsion.

**9.** Die Verwendung einer wie in irgendeinem der Ansprüche 1 bis 7 beanspruchten Verbindung zur Herstellung eines Medikaments zur Behandlung von Erkrankungen, Störungen, Zuständen oder Symptomen, die durch Zelladhäsion vermittelt werden.

**10.** Die Verwendung einer wie in irgendeinem der Ansprüche 1 bis 7 beanspruchten Verbindung zur Herstellung eines Medikaments zur Behandlung von Asthma.

**11.** Die Verwendung einer wie in irgendeinem der Ansprüche 1 bis 7 beanspruchten Verbindung zur Herstellung eines Medikaments zur Behandlung von allergischer Rhinitis.

**12.** Die Verwendung einer wie in irgendeinem der Ansprüche 1 bis 7 beanspruchten Verbindung zur Herstellung eines Medikaments zur Behandlung von multipler Sklerose.

**13.** Die Verwendung einer wie in irgendeinem der Ansprüche 1 bis 7 beanspruchten Verbindung zur Herstellung eines Medikaments zur Behandlung von Atherosklerose.

**14.** Die Verwendung einer wie in irgendeinem der Ansprüche 1 bis 7 beanspruchten Verbindung zur Herstellung eines Medikaments zur Behandlung von Entzündung.

**15.** Die Verwendung einer wie in irgendeinem der Ansprüche 1 bis 7 beanspruchten Verbindung zur Herstellung eines Medikaments zur Behandlung von entzündlicher Darmerkrankung.

**16.** Eine pharmazeutische Zusammensetzung, die eine wie in irgendeinem der Ansprüche 1 bis 7 beanspruchte Verbindung und einen pharmazeutisch annehmbaren Träger dafür enthält.

**Revendications**

**1.** Composé ayant la formule I :

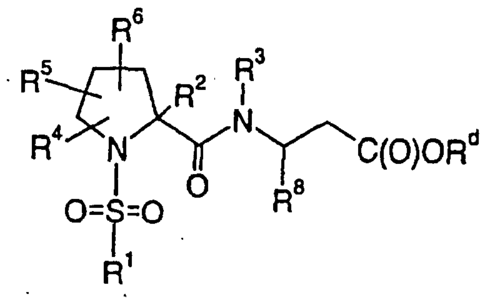

I

ou un sel pharmaceutiquement acceptable de celui-ci dans lequel :

$R^1$ est un groupe phényle éventuellement substitué par un à quatre substituants choisis indépendamment à partir de $R^b$ ;
$R^2$ est un d'hydrogène ou un alkyle en $C_{1-6}$
$R^3$ est

1) un d'hydrogène
2) un alkyle en $C_{1-10}$,
3) Cy, ou
4) un Cy-alkyle en $C_{1-10}$.

dans lequel l'alkyle est éventuellement substitué par un à quatre substituants choisis indépendamment à partir de $R^a$ ; et Cy est éventuellement substitué par un à quatre substituants choisis indépendamment à partir de $R^b$ ;
$R^4$, $R^5$ et $R^6$ sont chacun indépendamment choisis à partir du groupe composé de

1) un hydrogène, ou
2) un groupe choisi parmi Rb ;

$R^8$ est un groupe Cy-($Cy^1$) dans lequel Cy et $Cy^1$ sont chacun un phényle éventuellement substitué par un à quatre substituants choisis indépendamment parmi Rb ;
$R^a$ est

1) $-CF_3$,
2 ) $-OR^d$,
3) $-NO_2$,
4) un halogène,
5) $-S(O)_mR^d$,
6) $-SR^d$,
7) $-S(O)_2OR^d$,
8) $-S(O)_mNR^dR^e$,
9) $-NR^dR^e$,
10) $-O(CR^fR^g)_nNR^dR^e$,
11) $-C(O)R^d$,
12) $-CO_2R^d$,
13 ) $-CO_2(CR^fR^g)_nCONR^dR^e$,

14) $-OC(O)R^d$,
15) -CN,
16) $-C(O)NR^dR^e$,
17) $-NR^dC(O)R^e$,
18) $-OC(O)NR^dR^e$,
19) $-NR^dC(O)OR^e$,
20) $-NR^dC(O)Nr^dR^e$,
21) $-CR^d(N\text{-}OR^e)$, ou
22) Cy éventuellement substitué par un groupe choisi indépendamment parmi $R^c$ ;

$R^b$ est

1) un groupe choisi à partir de $R^a$,
2) un alkyle en $C_{1\text{-}10}$,
3) un alcényle en $C_{2\text{-}10}$,
4) un alcynyle en $C_{2\text{-}10}$, ou
5) un Cy-alkyle en $C_{1\text{-}10}$,

dans lesquels les alkyles, alcényles, alcynyles et Cy sont optionnellement substitués par un groupe choisi indépendamment parmi $R^c$ ;
$R^c$ est

1) un halogène,
2) CN,
3) NH(alkyle $C_{1\text{-}5}$),
4) N(alkyle $C_{1\text{-}5})_2$,
5) amino,
6) carboxy,
7) alkyle en $C_{1\text{-}4}$,
8) alcoxy en $C_{1\text{-}4}$,
9) aryle,
10) alkyle $C_{1\text{-}4}$ aryle, ou
11) aryloxy ;

$R^d$ et $R^e$ sont choisis indépendamment parmi un hydrogène, un alkyle en $C_{1\text{-}10}$, un alcényle en $C_{2\text{-}10}$, un alcynyle en $C_{2\text{-}10}$, Cy et un Cy-alkyle en $C_{1\text{-}10}$, dans lesquels les alkyles, alcényles, alcynyles et Cy sont optionnellement substitués par un à quatre substituants choisis indépendamment parmi $R^c$ ; ou
$R^d$ et $R^e$ de concert avec les atomes auxquels ils sont attachés forment un hétérocycle de 5 à 7 éléments contenant de 0 à 2 hétéroatomes supplémentaires choisis indépendamment parmi l'oxygène, le soufre et l'azote ;
$R^f$ et $R^g$ sont indépendamment choisis parmi un atome d'hydrogène, un alkyle en $C_{1\text{-}10}$, un Cy et un Cy-alkyle en $C_{1\text{-}10}$, ou
$R^f$ et $R^g$ de concert avec l'atome de carbone auquel ils sont attachés forment un cycle de 5 à 7 éléments contenant de 0 à 2 hétéroatomes choisis indépendamment parmi l'oxygène, le soufre et l'azote ;
Cy et $Cy^1$ sont

1) un cycloalkyle,
2) un hétérocyclyle,
3) un aryle, ou
4) un hétéroaryle ;

m est un entier de 1 à 2 ; et
n est un entier de 1 à 10.

**2.** Composé selon la revendication 1 dans lequel $R^1$ est un phényle optionnellement substitué par un à deux groupes choisis indépendamment parmi un halogène, un O-alkyle en $C_{1\text{-}3}$ et un trifluorométhyle.

**3.** Composé selon la revendication 1 ou la revendication 2 dans lequel $R^1$ est un 3,5-dichlorophényle ou 3-trifluorométhylphényle.

**4.** Composé selon l'une quelconque des revendications 1 à 3 dans lequel $R^8$ est un groupe biphényle éventuellement substitué, dans lequel les substituants éventuels sont un ou deux groupes indépendamment choisis parmi un halogène, un CN, un $OR^d$, un $O(CO)R^d$, un alkyle en $C_{1-5}$ éventuellement substitué par un ou deux groupes choisis parmi un $R^c$, un $CF_3$, et un $OC(O)NR^dR^e$.

**5.** Composé selon la revendication 1 ayant la formule Ib :

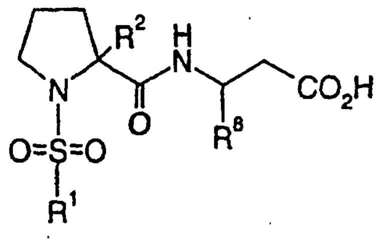

Ib

dans laquelle

$R^1$ est un groupe phényle éventuellement substitué par un à quatre substituants choisis indépendamment parmi $R^b$ ;

$R^2$ est un H ou un alkyle en $C_{1-6}$ ; et

$R^8$ est un groupe Cy-($Cy^1$) dans lequel Cy et $Cy^1$ sont chacun un groupe phényle éventuellement substitué par un à quatre substituants choisis indépendamment parmi $R^b$.

**6.** Composé selon la revendication 5 dans lequel

$R^1$ est un phényle éventuellement substitué par un ou deux groupes choisis parmi un halogène, un O-alkyle en $C_{1-3}$, et un groupe trifluorométhyle ;

$R_2$ est un H ou un méthyle ;

$R_8$ est un biphényle éventuellement substitué dans lequel les substituants éventuels sont un ou deux groupes choisis indépendamment parmi un halogène, un CN, un $OR^d$, un $OC(O)R^d$, un alkyle en $C_{1-5}$ éventuellement substitué avec un ou deux groupes choisis parmi un $R^c$, un $CF_3$, et un $OC(O)NR^dR^e$.

**7.** Composé selon l'une quelconque des revendications 1 à 5 choisi dans le groupe composé de :

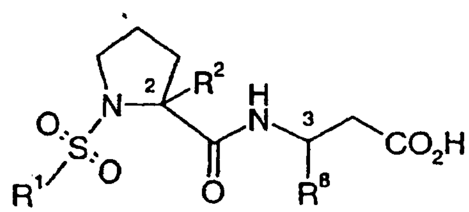

| 2/3* | $R^1$ | $R^2$ | $R^8$ |
|---|---|---|---|
| S/R | 3,5-diCl-Ph | H | Biphényle |
| S/S | 3,5-diCl-Ph | H | Biphényle |
| S/R | 3,5-diCl-Ph | H | 2'-méthoxybiphényle |
| S/S | 3,5-diCl-Ph | H | 2'-méthoxybiphényle |

*configuration stérique aux positions indiquées

(suite)

| 2/3* | $R^1$ | $R^2$ | $R^8$ |
|---|---|---|---|
| S/R | 3,5-diCl-Ph | H | 2'-cyanobiphényle |
| S/R | 3,5-diCl-Ph | H | 2'-formylbiphényle |
| S/R | 3,5-diCl-Ph | H | 2'-diméthylaminométhylbiphényle |
| S/R | 3,5-diCl-Ph | H | 2'-hydroxyméthylbiphényle |
| S/R | Ph | H | 2'-méthoxybiphényle |
| S/R | Ph | $CH_3$ | 2'-méthoxybiphényle |
| S/R | 3,5-diCl-Ph | $CH_3$ | 2'-méthoxybiphényle |
| S/R | Ph | $CH_3$ | 4'-flurobiphényle |
| S/R | 3,5-diCl-Ph | H | 4'-flurobiphényle |
| S/R | 3,5-diCl-Ph | H | 2'-$CF_3O$-biphényle |
| S/R | 3,5-diCl-Ph | $CH_3$ | 2'-$CF_3O$-biphényle |
| S/R | 3,5-diCl-Ph | $CH_3$ | 3'-méthoxybiphényle |
| S/R | 3,5-diCl-Ph | H | 3'-méthoxybiphényle |
| S/R | 3,5-diCl-Ph | $CH_3$ | 2'-méthoxy-3'-F-biphényle |
| S/R | 3,5-diCl-Ph | H | 2'-méthoxy-3'-F-biphényle |
| S/R | 3,5-diCl-Ph | $CH_3$ | 3'-méthoxy-2'-F-biphényle |
| S/R | 3,5-diCl-Ph | H | 3'-méthoxy-2'-F-biphényle |
| S/R | 3,5-diCl-Ph | $CH_3$ | 2'-méthoxy-5'-F-biphényle |
| S/R | 3,5-diCl-Ph | H | 2'-méthoxy-5'-F-biphényle |
| S/R | 3,5-diCl-Ph | $CH_3$ | 3'-méthoxy-5'-F-biphényle |
| S/R | 3,5-diCl-Ph | H | 3'-méthoxy-5'-F-biphényle |
| S/R | 3,5-diCl-Ph | $CH_3$ | 2'-méthoxy-6'-F-biphényle |
| S/R | 3,5-diCl-Ph | H | 2'-méthoxy-6'-F-biphényle |
| S/R | 3-Cl-Ph | $CH_3$ | 2'-méthoxybiphényle |
| S/R | 3,5-diCl-Ph | $CH_3$ | 2'-$CF_3O$-4'-F-biphényle |
| S/R | 3,5-diCl-Ph | H | 2'-$CF_3O$-4'-F-biphényle |
| S/R | 3,5-diCl-Ph | H | 2'-méthoxy-4'-F-biphényle |
| S/R | 3,5-diCl-Ph | $CH_3$ | 2'-méthoxy-4'-F-biphényle |
| S/R | 3,5-diCl-Ph | H | 3-(2'-méthoxyphényl)-phényle |
| S/R | 3,5-diCl-Ph | H | 4-(2'-cyclopropoxy)-biphényle |
| S/R | 3,5-diCl-Ph | $CH_3$ | 4-(2'-cyclopropoxy)-biphényle |

*configuration stérique aux positions indiquées

**8.** Utilisation d'un composé selon l'une quelconque des revendications 1 à 7 pour la fabrication d'un médicament pour inhiber l'adhérence des cellules.

**9.** Utilisation d'un composé selon l'une quelconque des revendications 1 à 7 pour la fabrication d'un médicament pour le traitement des maladies, désordres, états ou symptômes facilités par une adhérence des cellules.

**10.** Utilisation d'un composé selon l'une quelconque des revendications 1 à 7 pour la fabrication d'un médicament

pour le traitement de l'asthme.

**11.** Utilisation d'un composé selon l'une quelconque des revendications 1 à 7 pour la fabrication d'un médicament pour le traitement d'une rhinite allergique.

**12.** Utilisation d'un composé selon l'une quelconque des revendications 1 à 7 pour la fabrication d'un médicament pour le traitement d'une sclérose multiple.

**13.** Utilisation d'un composé selon l'une quelconque des revendications 1 à 7 pour la fabrication d'un médicament pour le traitement de l'athérosclérose.

**14.** Utilisation d'un composé selon l'une quelconque des revendications 1 à 7 pour la fabrication d'un médicament pour le traitement d'une inflammation.

**15.** Utilisation d'un composé selon l'une quelconque des revendications 1 à 7 pour la fabrication d'un médicament pour le traitement d'une affection abdominale inflammatoire.

**16.** Composition pharmaceutique qui comprend un composé selon l'une quelconque des revendications 1 à 7 et un vecteur pharmaceutique acceptable de celui-ci.